# DEMANDE DE BREVET EUROPEEN

(11) **EP 4 421 521 A1**
(43) Date de publication de la demande: **28.08.2024**
(21) Numéro de dépôt: 24154860.1
(22) Date de dépôt: 30.01.2024
(51) Int. Cl.: G01S 7/52, B06B 1/02

(54) **PROCÉDÉ ET SYSTÈME DE PILOTAGE D'UN DISPOSITIF ÉMETTEUR D ONDES**

(30) Priorité: 27.02.2023 FR 2301783
(71) Demandeur: SUPERSONIC IMAGINE, 13290 Aix-en-Provence (FR)
(72) Inventeur: Cordonnier, Hubert, 13290 Aix-en-Provence (FR); Martin, Jean-Alain, 13290 Aix-en-Provence (FR); Roux, Guillaume, 13290 Aix-en-Provence (FR)
(74) Mandataire: RVDB Nantes

(57) **Abrégé**

La présente divulgation concerne un procédé et un système (100 ; 200) de pilotage d'un dispositif émetteur (20) comprenant un transducteur (22) pour émettre des ondes (W1), ledit procédé comprenant une modulation de signaux de sortie (SG1) émis depuis un pulseur (101) pour piloter le transducteur (22), la modulation étant réalisé par un contrôleur (22) selon au moins l'un parmi : un contrôle d'une tension d'alimentation (V+, V-) du pulseur (101) par un contrôleur d'alimentation (122a, 122b) connecté à une borne d'alimentation (SHV+, SHV-, GND1) du pulseur ; et un contrôle de signaux de retour (SG2) du transducteur (22) par un contrôleur de retour (126) connecté sur une borne de retour (OUT2) du transducteur (22).

## Description

### Technique antérieure

Un dispositif émetteur d'ondes comprend habituellement une pluralité d'éléments transducteurs (par exemple disposés en réseau) à des fins de communication, d'imagerie ou de balayage, par exemple dans le domaine de l'imagerie, en particulier l'imagerie médicale, du radar, du sonar, de la sismologie, des communications sans fil, de la radioastronomie, de l'acoustique ou de la biomédecine. Un exemple comprend l'imagerie par ultrasons.

A cet effet, le dispositif émetteur d'ondes peut être piloté au moyen de signaux électriques émis par un ou des pulseurs. Ces signaux définissent des ondes transmises à des éléments transducteurs du dispositif émetteur, causant l'émission d'ondes dans un milieu donné. Des signaux électriques peuvent être récupérés en retour par les éléments transducteurs, ces même transducteurs ou d'autres transducteurs, ces signaux représentant une réponse (ou écho) du milieu aux sollicitations ondulatoires.

Par exemple, l'imagerie ultrasonore peut avoir pour but d'estimer la réflectivité d'un milieu. La fréquence des signaux peut alors être choisie en fonction des caractéristiques du milieu observé (par exemple des tissus humains).

Plus particulièrement, dans un procédé conventionnel d'imagerie par ultrasons, on peut utiliser par exemple un dispositif transducteur d'ultrasons équipé d'au moins un élément transducteur d'ultrasons pour convertir les signaux électriques en ondes ultrasonores. Chaque transducteur peut ainsi émettre un ou successivement plusieurs faisceaux ultrasonores en direction d'un milieu, ce qui correspond à une opération d'émission. Ensuite, dans une opération de réception, un ensemble de signaux d'écho rétrodiffusés peuvent être reçus du milieu par le même ensemble ou par un autre ensemble d'éléments transducteurs. En particulier, chacun des éléments transducteurs peut par exemple convertir un signal d'écho reçu en un signal électrique. Le signal peut ensuite être traité par le système à ultrasons ou par tout système associé (dédié), directement connecté ou non. Par exemple, le signal peut être amplifié, filtré, numérisé et/ou une opération de conditionnement du signal peut être effectuée. Les éléments transducteurs peuvent être disposés selon une ligne de transducteurs, une matrice, ou comme un réseau de transducteurs ou toute autre configuration.

Il a cependant été constaté que certains dispositifs émetteurs d'ondes, notamment des systèmes d'imagerie (de type imagerie par ultrasons ou autre), souffrent d'un problème de vieillissement accéléré, qui peut se traduire par l'occurrence de défauts, de dégradation du système et/ou de mauvaises performances du système. Ces problèmes de défauts ou de dérive des performances peuvent conduire à diverse défaillances, notamment à une qualité d'image dégradée dans le cas d'applications d'imagerie (par exemple d'imagerie ultrasonore).

En outre, la mise au point de tels dispositifs émetteurs d'ondes est souvent complexe ce qui peut rendre la conception et/ou calibration de tels dispositifs particulièrement compliquées et coûteuses.

### Exposé de la divulgation

L'un des objets de la présente divulgation est de résoudre au moins l'un des problèmes ou déficiences décrits précédemment.

En particulier, il peut être souhaitable d'améliorer la fiabilité et/ou les performances d'un dispositif émetteur d'ondes, notamment mais pas exclusivement d'un dispositif transducteur d'ultrasons.

En particulier, il peut être souhaitable d'améliorer le pilotage d'un dispositif émetteur d'ondes, notamment mais pas exclusivement d'un dispositif transducteur d'ultrasons.

A cet effet, selon un premier aspect, la présente divulgation vise un procédé pour piloter un dispositif émetteur d'ondes comprenant un transducteur pour émettre des ondes. Le procédé selon le premier aspect comprend :
- modulation de signaux de sortie émis depuis un pulseur pour piloter le transducteur, ladite modulation étant réalisé par un contrôleur selon au moins l'un parmi :
   a) un contrôle d'une tension d'alimentation du pulseur par un contrôleur d'alimentation connecté à une borne d'alimentation du pulseur ; et
   c) un contrôle de signaux de retour du transducteur par un contrôleur de retour connecté sur une borne de retour du transducteur.

En fournissant un tel procédé, il devient avantageusement possible d'améliorer le pilotage du dispositif émetteur d'ondes et donc les performances et la fiabilité du système dans son ensemble. La modulation permet en particulier de mieux contrôler les signaux de sortie envoyés au transducteur et ainsi de s'assurer que les valeurs limites théoriques des paramètres (par exemple en courant, tension et/ou puissance) du transducteur, et plus généralement de la chaîne de transmission depuis le pulseur au transducteur, sont respectées. En maîtrisant plus précisément le pilotage du transducteur, on peut éviter de stresser excessivement le système, limiter les défaillances (pannes, dégradations, etc.), assurer une bonne fiabilité du système et garantir que le système présente des performances satisfaisantes.

Grâce au concept de la présente divulgation, il est possible d'adapter les ondes envoyées avec plus de flexibilité (en puissance, fréquence, durée, etc.) sans détériorer les pulseurs ou transducteurs, ce qui permet de s'affranchir de certains compromis ou limitations (notamment en termes de puissance) avec lesquels il faut habituellement composer avec ce type de dispositif pour garantir de bonnes performances et un vieillissement normal de l'ensemble. Contrairement aux systèmes classiques, il est possible grâce au concept de la présente divulgation de configurer le dispositif de sorte à émettre les ondes désirées, c'est-à-dire conformes aux caractéristiques d'ondes souhaitées selon l'application visée.

Avec une modification limitée de l'ensemble, et donc un faible surcoût, on peut ainsi obtenir un dispositif émetteur d'ondes présentant des capacités de haut niveau.

En mettant en l'oeuvre le concept de la présente divulgation, on peut par exemple améliorer la qualité d'image, telle que par exemple la qualité d'une image ultrasonore, dans une application d'imagerie ultrasonore. Grâce au procédé de la présente divulgation, il est notamment possible d'obtenir une bonne qualité d'image.

Le procédé selon la divulgation peut comporter d'autres caractéristiques qui peuvent être prises séparément ou en combinaison, notamment parmi les modes de réalisation qui suivent qui sont présentés à titre d'illustration uniquement et peuvent être combinés ou associés sauf stipulation contraire.

Selon un exemple, au cours du contrôle a), le contrôleur d'alimentation module une puissance délivrée au pulseur sur la borne d'alimentation à partir d'une tension statique délivrée par un régulateur de tension.

Selon un exemple, la borne d'alimentation du pulseur à laquelle est connectée le contrôleur d'alimentation est l'une quelconque parmi : une borne d'alimentation positive, une borne d'alimentation négative et une borne de masse du pulseur.

Selon un exemple, la modulation est réalisée au moyen d'un (ou d'au moins un) dit contrôleur d'alimentation, la modulation étant configurée pour causer une variation au cours du temps de la tension d'alimentation du pulseur selon au moins deux valeurs non nulles distinctes.

Selon un exemple, au cours du contrôle b), le contrôleur de sortie module au moins l'une des caractéristiques électriques des signaux de sortie du pulseur parmi la tension, le courant et la puissance desdits signaux de sortie.

Selon un exemple, la borne de retour du transducteur est connectée en série avec une masse du dispositif émetteur.

Selon un exemple, la modulation des signaux de sortie est réalisée à une fréquence de modulation Fm supérieure ou égale à une fraction 1/P de la fréquence de tir Ft à laquelle le transducteur émet des ondes en réponse au signaux de sortie du pulseur, P étant un entier égal à 10.

Selon un exemple, le dispositif émetteur comprend une pluralité de transducteurs pilotés chacun par un pulseur respectif, la modulation de signaux étant réalisé indépendamment par canal entre chaque transducteur et le pulseur respectif.

Selon un exemple, le procédé comprend :
- mesure d'au moins un paramètre de fonctionnement d'une chaîne de transmission au travers de laquelle le pulseur envoie les signaux de sortie au transducteur ;
dans lequel la modulation des signaux de sortie est réalisée en fonction dudit au moins paramètre de fonctionnement mesuré.

Selon un exemple, ledit au moins un paramètre de fonctionnement comprenant au moins l'un parmi :
- une tension en sortie du pulseur ;
- la tension d'alimentation du pulseur ;
- une tension de masse du pulseur ;
- une tension aux bornes du dispositif émetteur ;
- une tension en réception du transducteur ;
- un courant en sortie du pulseur ;
- un courant d'alimentation du pulseur ;
- un courant de masse du pulseur ;
- un courant circulant dans le dispositif émetteur ;
- une température du pulseur ;
- une température du dispositif émetteur ;
- un champ électrique émis par le pulseur ;
- un champ électrique émis par le dispositif émetteur ;
- un champ magnétique émis par le pulseur ;
- un champ magnétique émis par le dispositif émetteur ;
- une pression acoustique émise par le dispositif émetteur ; et
- une pression acoustique reçue par le dispositif émetteur.

Selon un exemple, le procédé comprend :
- comparaison dudit au moins un paramètre de fonctionnement avec respectivement une valeur seuil ;
la modulation des signaux de sortie étant fonction d'un résultat de ladite comparaison.

Selon un exemple, le contrôleur d'alimentation régule la tension d'alimentation à une première valeur au cours du contrôle a) si ledit au moins un paramètre de fonctionnement n'excède pas une valeur seuil respective, et
dans lequel le contrôle a) comprend une adaptation de la tension d'alimentation depuis la première valeur vers une deuxième valeur, différente de la première valeur, si ledit au moins un paramètre de fonctionnement excède ladite valeur seuil respective.

Selon un exemple, la modulation des signaux de sortie est réalisée par asservissement de l'un au moins parmi les contrôles a), b) et c) en fonction dudit au moins un paramètre de fonctionnement mesuré.

Selon un exemple, la mesure dudit au moins un paramètre de fonctionnement est réalisée au cours d'une calibration de la chaîne de transmission,
dans lequel la modulation est réalisée après la calibration en réponse à au moins une commande déterminée en fonction dudit au moins un paramètre de fonctionnement.

Selon un exemple, le procédé comprend :
- mesure d'au moins un paramètre de fonctionnement de chaînes de transmission au travers desquelles chaque traducteur reçoit des signaux de sortie d'un pulseur respectif ; et
- sur détection qu'un paramètre de fonctionnement d'une chaîne de transmission satisfait un critère prédéfini, adaptation de la modulation des signaux de sortie émis vers le transducteur de ladite chaîne de transmission.

Selon un exemple, la modulation des signaux de sortie est réalisée en fonction d'au moins l'un parmi :
- un mode d'imagerie implémenté par le pulseur pour piloter le dispositif émetteur ;
- un type du dispositif émetteur ; et
- des paramètres utilisateurs du dispositif émetteur.

Selon un exemple, le transducteur est un transducteur piézoélectrique.

Selon un exemple, ledit procédé est appliqué à l'imagerie médicale par ultrasons.

Selon un deuxième aspect, la présente divulgation peut impliquer un programme d'ordinateur comprenant des instructions qui, lorsque le programme est exécuté par un ordinateur ou plus précisément par un système de pilotage pilotant un dispositif transducteur, participe à la mise en oeuvre du procédé selon le premier aspect. En particulier, les différentes étapes du procédé selon le premier aspect peuvent faire intervenir des instructions de programmes d'ordinateurs.

Un tel programme d'ordinateur peut utiliser n'importe quel langage de programmation ou équivalent, et il peut se trouver sous la forme d'un code source, d'un code objet, ou d'un code intermédiaire entre un code source et un code objet, tel que dans une forme partiellement compilée, ou sous n'importe quelle autre forme souhaitable.

Selon un troisième aspect, la présente divulgation concerne un support d'enregistrement (ou support d'informations), lisible par un ordinateur (ou un processeur), sur lequel est enregistré un programme d'ordinateur selon le troisième aspect de la présente divulgation.

D'une part, le support d'enregistrement peut être n'importe quel entité ou dispositif capable de stocker le programme, telle qu'au moins une mémoire volatile et/ou non volatile. Par exemple, le support peut comporter un moyen de stockage, tel qu'une mémoire non-volatile réinscriptible, une mémoire ROM, un CD-ROM ou une mémoire ROM de type circuit microélectronique, ou encore un moyen d'enregistrement magnétique ou un disque dur. Cette mémoire peut par exemple comprendre une mémoire de carte graphique (ou carte vidéo), ce type de mémoire étant notamment apte à traiter des données d'image (ou données vidéo).

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon la présente divulgation peut être en particulier téléchargé grâce à un réseau filaire ou non, de type local ou non (Bluetooth^{®} par exemple, Wi-Fi, Ethernet, Internet, 4G, 5G ou autres).

Alternativement, le support d'enregistrement peut être un circuit intégré dans lequel le programme d'ordinateur est incorporé, le circuit intégré étant adapté pour exécuter ou pour être utilisé dans l'exécution du procédé en question.

Selon un quatrième aspect, la présente divulgation concerne un système de pilotage configuré pour piloter un dispositif émetteur d'ondes comprenant un transducteur pour émettre des ondes, et ce en mettant en oeuvre le procédé de pilotage du premier aspect de la présente divulgation.

Selon un exemple, le système de pilotage comprend une mémoire associée à un processeur, cette mémoire comprenant un programme d'ordinateur selon le deuxième aspect de la divulgation.

Selon un exemple, le système de pilotage comprend :
- un pulseur ; et
- un contrôleur configuré pour moduler des signaux de sortie émis depuis le pulseur pour piloter le dispositif émetteur, ledit contrôleur comprenant au moins l'un parmi :
   a) un contrôleur d'alimentation, connecté à une borne d'alimentation du pulseur, configuré pour contrôler une tension d'alimentation du pulseur ; et
   c) un contrôleur de retour, connecté à une borne de retour du transducteur, configuré pour contrôler des signaux de retour du transducteur.

Le système de pilotage peut avoir des fonctionnalités qui correspondent aux opérations du procédé selon le premier aspect de la divulgation. En particulier, les différents modes de réalisation mentionnés ci-avant en relation avec le procédé selon le premier aspect de la divulgation ainsi que les avantages associés peuvent s'appliquer de façon analogue au système de pilotage selon le quatrième aspect de la présente divulgation.

Les caractéristiques et avantages de la divulgation apparaitront plus précisément à la lecture de la description qui va suivre, donnée uniquement à titre d'exemple non limitatif, et faite en référence aux figures annexées. Notamment, les exemples illustrés dans les figures peuvent être combinés entre eux, sauf incohérence notoire.

### Brève description des figures

D'autres caractéristiques et avantages de la présente divulgation ressortiront de la description des exemples de réalisation non limitatifs de la présente divulgation ci-après, en référence aux figures 1 à 15 annexées, sur lesquelles :
[Fig. 1] montre un dessin schématique d'un système de pilotage d'un dispositif émetteur d'ondes, selon des exemples de réalisation de la présente divulgation ;
[Fig. 2] montre un dessin schématique d'un système de pilotage d'un dispositif émetteur d'ondes, selon des exemples de réalisation de la présente divulgation ;
[Fig. 3] montre un dessin schématique d'un système d'imagerie ultrasonore selon un exemple de réalisation de la présente divulgation ;
[Fig. 4] montre un dessin schématique d'un système de pilotage d'un dispositif transducteur ultrasonore selon des exemples de réalisation de la présente divulgation ;
[Fig. 5] illustre schématiquement un contrôleur configuré pour moduler les signaux de sortie d'un pulseur du système de la figure 4, selon des exemples de réalisation de la présente divulgation ;
[Fig. 6] est un diagramme illustrant schématiquement un procédé de pilotage mettant en oeuvre le système de pilotage de la figure 4, selon des exemples de réalisation de la présente divulgation ;
[Fig. 7] illustre schématiquement la modulation de signaux de sortie d'un pulseur du système de pilotage de la figure 4, selon des exemples de réalisation de la présente divulgation ;
[Fig. 8] illustre schématiquement la modulation de signaux de sortie d'un pulseur du système de pilotage de la figure 4, selon des exemples de réalisation de la présente divulgation ;
[Fig. 9] illustre schématiquement la modulation de signaux de sortie d'une pluralité de pulseurs du système de pilotage de la figure 4, selon des exemples de réalisation de la présente divulgation ;
[Fig. 10] montre un dessin schématique d'un système de pilotage d'un dispositif transducteur ultrasonore selon des exemples de réalisation de la présente divulgation ;
[Fig. 11] est un diagramme illustrant schématiquement un procédé de pilotage mettant en oeuvre le système de pilotage de la figure 10, selon des exemples de réalisation de la présente divulgation ;
[Fig. 12] illustre schématiquement des moyens de mesure d'un courant dans le système de pilotage de la figure 10, selon des exemples de réalisation de la présente divulgation ;
[Fig. 13] illustre schématiquement des moyens de mesure d'un courant dans le système de pilotage de la figure 10, selon des exemples de réalisation de la présente divulgation ;
[Fig. 14] illustre schématiquement des moyens de mesure d'une tension dans le système de pilotage de la figure 10, selon des exemples de réalisation de la présente divulgation ; et
[Fig. 15] illustre schématiquement des moyens de mesure d'une puissance dans le système de pilotage de la figure 10, selon des exemples de réalisation de la présente divulgation.

### Description des modes de réalisation

La présente divulgation porte sur des systèmes de pilotage d'un dispositif émetteur d'ondes et des procédés de pilotage d'un tel dispositif émetteur. Ce dispositif émetteur peut constituer un dispositif à ultrasons, bien que d'autres formes soient possibles. Plus particulièrement, il peut être un dispositif d'imagerie, par exemple un dispositif d'imagerie médical, tel que par exemple un dispositif (ou système) d'échographie médical.

Un dispositif émetteur d'ondes, tel qu'un dispositif transducteur par exemple, peut être piloté au moyen de signaux électriques émis par un ou des pulseurs électroniques, dits plus simplement « pulseurs », c'est à dire des générateurs de signaux électriques. Chaque transducteur (par exemple de type ultrasonore) du dispositif émetteur peut ainsi être piloté par les signaux délivrés par un pulseur d'un système de pilotage. La transmission de ces signaux électriques peut être assurée par une chaîne de transmission depuis les pulseurs vers les transducteurs du dispositif émetteur.

Or, il a été constaté que le pilotage d'un tel dispositif émetteur, par exemple dans une application d'imagerie (par ultrasons par exemple), n'est pas toujours bien maîtrisé. En effet, les signaux émis par le ou les pulseurs ne respectent pas toujours les limites prévues pour chaque composant du système. Il a été observé que les valeurs limites fixées pour certains paramètres (courant, tension, température, etc.) de composants dans la ou les chaînes de transmission du système peuvent être dépassées ou non respectées, ce qui peut conduire à une dégradation ou dérive des performances du système et/ou à un vieillissement prématuré de celui-ci.

Ces contraintes et/ou problèmes de performances et de fiabilité peuvent avoir plusieurs origines. En premier lieu, les pulseurs sont des composants configurés pour générés des signaux de relativement haute tension (par exemple de l'ordre de ± 100V) et de relativement haut courant (par exemple de l'ordre de ± 2A). Dans certains cas, il a été observé qu'un pulseur peut générer en sortie des signaux trop élevés en tension et/ou courant, ce qui peut dégrader le système (par exemple causer une montée en température et une panne du pulseur) ou causer un vieillissement prématuré du système. D'autres éléments du système peuvent également se détériorer en raison du stress engendré par ces dépassements en tension et/ou courant.

Par ailleurs, dans certains systèmes d'imagerie par ultrasons, les transducteurs piézoélectriques du dispositif émetteur sont pilotés en transmission par des pulseurs générant des signaux pouvant contenir des harmoniques, en particulier lorsque ces signaux sont de forme carrée. Or, ces harmoniques ne sont généralement pas (ou peu) converties en énergie acoustique par les transducteurs piézoélectriques et cause de la dissipation thermique, ce qui limite le rendement énergétique du système dans la mesure où certaines parties des signaux émis par les pulseurs sollicitent le système sans être utiles pour le pilotage du transducteur. Ainsi, l'énergie non utilisée transitant dans un pulseur peut dégrader les performances thermiques et électriques du pulseur. L'énergie non utilisée rayonnée par le système peut en outre dégrader les performances électromagnétiques du système. Enfin, l'énergie non utilisée convertie en chaleur par le transducteur peut dégrader les performances thermiques du transducteur.

De plus, une partie des harmoniques peut être convertie par le transducteur et se retrouver dans les ondes émises dans le milieu extérieur. Dans certaines applications, la présence de telles harmoniques n'est pas souhaitable, y compris dans le domaine acoustique. L'émission de ces harmoniques peut avoir pour conséquence que les signaux acoustiques émis par le système sont différents des signaux théoriques visés, ce qui peut conduire notamment à une dégradation de la qualité d'image dans le cas d'une application d'imagerie (d'imagerie par ultrasons par exemple).

Dans certains cas, l'émission de telles harmoniques conduit à des rayonnements électromagnétiques excessifs, ce qui peut poser problème notamment lorsqu'une limite maximale de ces rayonnements doit être respectée (par exemple conformément à une réglementation imposée pour obtenir une certification du système).

Pour limiter les harmoniques et éviter les rayonnements électromagnétiques trop importants, il est connu d'utiliser des pulseurs linéaires capables de générer des signaux sinusoïdaux, mais cette technique est coûteuse et présente une efficacité peu adaptée, en particulier au domaine médical, notamment en raison du fait que les ondes générées sont limitées en puissance et produisent beaucoup de chaleur.

Les contraintes et/ou problèmes mentionnés précédemment imposent donc de réaliser des compromis ou de limiter certaines caractéristiques d'un tel dispositif émetteur d'ondes afin de garantir de bonnes performances et un vieillissement acceptable, voire performant selon les exigences du marché, de l'ensemble. Ces contraintes et/ou problèmes peuvent en outre rendre difficile la qualification d'un tel dispositif émetteur d'ondes selon des normes en vigueur.

La présente divulgation permet avantageusement notamment de résoudre les problèmes et déficiences mentionnés ci-avant grâce à l'introduction d'un ou plusieurs contrôleurs dans un système de pilotage d'un dispositif émetteur d'ondes, ce ou ces contrôleurs permettant de moduler les signaux de sortie d'un ou plusieurs pulseurs, et ce de façon dynamique, par exemple en temps réel (ou quasi-temps réel). Contrairement aux systèmes de pilotage conventionnels, la présente divulgation permet un contrôle précis et flexible au cours du temps des signaux émis en sortie d'un pulseur vers un transducteur du dispositif émetteur.

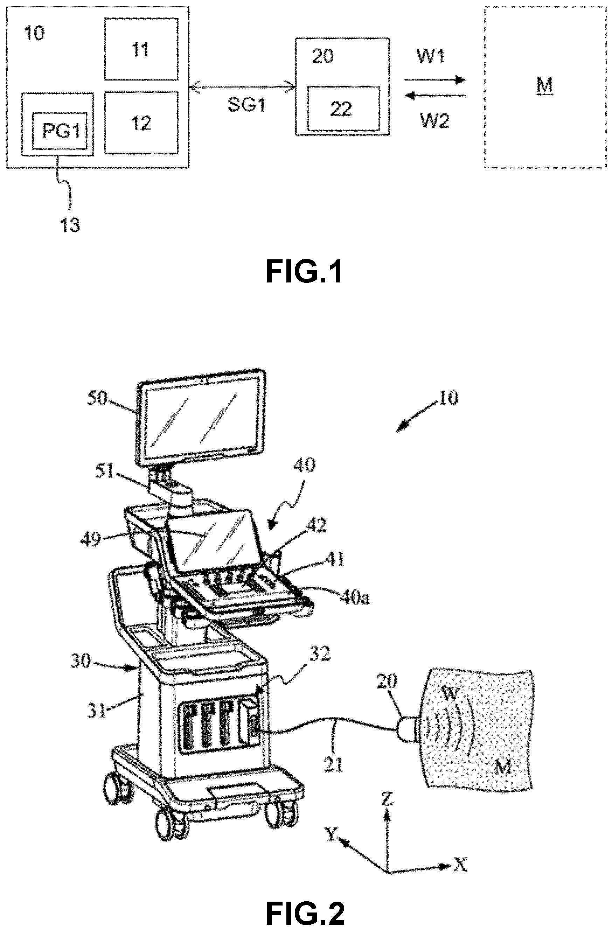

Des procédés de pilotage et des systèmes de pilotage pour piloter un dispositif émetteur d'ondes vont être décrits dans ce qui va suivre selon des modes particuliers de réalisation de la divulgation en référence conjointement aux figures 1-15. Sauf indications contraires, les éléments communs ou analogues à plusieurs figures portent les mêmes signes de référence et présentent des caractéristiques identiques ou analogues, de sorte que ces éléments communs ne sont généralement pas à nouveau décrits par souci de concision.

Les termes « premier(s) » (ou première(s)), « deuxième(s) », etc.) sont utilisés dans ce document par convention arbitraire pour permettre d'identifier et de distinguer différents éléments (tels que des opérations, des dispositifs, etc.) mis en oeuvre dans les modes de réalisation décrits ci-après.

Comme précédemment indiqué, la présente divulgation vise notamment un procédé de pilotage d'un dispositif émetteur d'ondes. La figure 1 représente schématiquement un système (ou dispositif) 10, dit aussi système de pilotage ou système de commande, configuré pour piloter un dispositif 20 émetteur d'ondes selon des exemples de réalisation de la présente divulgation.

Le dispositif 20 est configuré pour émettre, et éventuellement aussi recevoir, des ondes W. La nature de ces ondes W dépend de la nature et de la configuration du dispositif 20, au vu notamment de l'utilisation qui en est faite. Selon un exemple, le dispositif 20 est configuré pour émettre des ondes W1. Selon un autre exemple, le dispositif 20 est configuré pour émettre des ondes W1 et recevoir des ondes W2.

Les ondes W peuvent par exemple être (ou comprendre) des ondes acoustiques, par exemple de type ultrasonore. À titre d'exemple, on considère par la suite que le dispositif 20 est un dispositif transducteur à ultrasons (ou sonde à ultrasons) configuré pour émettre des ondes ultrasonores W1. À noter toutefois que d'autres exemples de dispositif émetteurs d'ondes sont possibles selon la présente divulgation comme précisé ultérieurement. En particulier, tout ou partie du dispositif émetteur d'ondes de la présente divulgation peut être mis en oeuvre dans une sonde, par exemple une sonde à ultrasons. Autrement dit, tout ou partie du dispositif de la divulgation peut être externe à la sonde.

Le système de pilotage 10 peut comprendre dans cet exemple une unité (ou module) de traitement 11 et une unité (ou module) de commande 12.

L'unité de commande 12 (dite aussi unité de commande de puissance) est configurée pour piloter le dispositif transducteur 20 au moyen de signaux électriques SG1 qui sont échangés (ou transmis) entre le système 10 et le dispositif transducteur 20. L'unité de commande 12 génère ainsi des signaux électriques SG1 qui sont transmis au dispositif transducteur 20 pour causer l'émission, par ledit dispositif transducteur 20, d'ondes ultrasonores W1 en direction et/ou dans un milieu M. Les signaux électriques SG1 ainsi générés sont représentatifs des (ou définissent les) ondes ultrasonores W1 projetées dans le milieu M. À cette fin, l'unité de commande 12 peut par exemple être ou comprendre au moins un pulseur électronique, dit aussi « pulseur ». Les signaux électriques SG1 peuvent être de diverses formes, par exemple de forme carrée, sinusoïdale, aléatoire (ou pseudo-aléatoire), quelconque, etc.

L'unité de commande 12 peut éventuellement comprendre des dispositifs récepteurs ou circuits récepteurs (non représentés) configurés pour recevoir des signaux électriques SG1 en provenance du dispositif transducteur 20.

L'unité de traitement 11 peut être configurée pour commander l'unité de commande 12, par exemple en commandant les signaux électriques SG1. Cette unité de traitement 11 peut par exemple être ou comprendre au moins un processeur.

Selon un exemple, l'unité de traitement 11 et/ou l'unité de pilotage 12 sont comprises dans le corps 31 (élément central) du système 10 représenté à titre illustratif en figure 2.

Plus particulièrement, l'unité de traitement 11 peut être configurée pour contrôler les signaux électriques SG1 qui sont générés par l'unité de commande 12. L'unité de traitement 11 peut également être configurée pour traiter (et/ou interpréter) des signaux électriques SG1 éventuellement reçus par l'unité de commande 12 en provenance du dispositif transducteur 20. Ces signaux SG1 sont représentatifs d'ondes W2 reçues par le dispositif transducteur 20 en provenance du milieu M (figure 1). Ces ondes W2 forment par exemple un ou plusieurs échos ultrasonores, c'est-à-dire une réponse du milieu M aux ondes ultrasonores W1 émises en direction dudit milieu. Le traitement réalisé par l'unité de traitement 11 sur les signaux SG1 reçus peut varier selon le cas et comprendre par exemple au moins l'une quelconque parmi des opérations d'amplification, de filtrage, de numérisation et de conditionnement des signaux SG1.

Le système 10 peut comprendre le dispositif transducteur 20. En variante, le dispositif transducteur 20 peut être externe au système 10. Par exemple, le dispositif transducteur 20 peut être connecté au système 10 par un câble ou peut communiquer sans fil avec lui. Dans ce dernier cas, le dispositif transducteur 10 peut par exemple comprendre une batterie et recevoir des signaux de communication du système 10 qui représentent les signaux électriques SG1 (par exemple les fréquences de pilotage et/ou toute information comprise dans les signaux électriques). Le dispositif transducteur 20 peut alors reproduire les signaux électriques SG1 en interne à partir des signaux de communication reçus.

Le dispositif transducteur 20 peut par exemple être un dispositif émetteur d'ondes conventionnel. Ainsi, une différence selon la présente divulgation peut résider dans la manière dont le dispositif transducteur 20 est piloté et les moyens associés qui sont mis en oeuvre pour réaliser un tel pilotage. Selon une variante, le dispositif transducteur 20 comprend un contrôleur comme décrit ci-après dans des exemples.

Le système 10 peut être stationnaire ou mobile. Le dispositif transducteur 20 peut également être stationnaire ou mobile. Par exemple, le système 10 peut être un système fixe (par exemple comprenant une unité de traitement et un dispositif d'affichage, comme décrit ci-dessous) et le dispositif transducteur 20 peut être mobile (par exemple un dispositif capteur, un dispositif de mesure, ou une sonde). Toutefois, il est également possible que le dispositif transducteur 20 soit intégré au système 10, et que le système 10 soit un système mobile. Par exemple, le système 10 peut être configuré pour être piloté de manière autonome, par exemple grâce à une batterie incluse. D'autres exemples sont décrits ci-dessous.

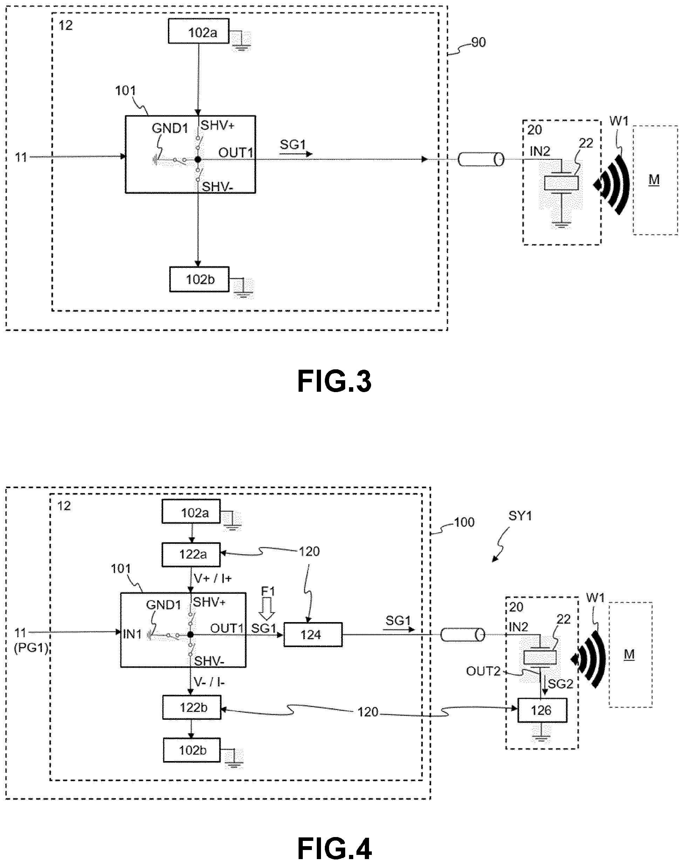

Selon un exemple, le système 10 peut comprendre au moins une mémoire 13 utilisée par l'unité de traitement 11 pour commander l'unité de commande 10. Cette mémoire peut éventuellement faire partie de l'unité de traitement 11. Dans des exemples, le processeur et la mémoire de l'unité de traitement 11 peuvent être incorporés dans le système 10 illustré en figure 3 ou peuvent être incorporés dans un ordinateur ou un dispositif informatique lié de manière communicante à celui-ci. La mémoire peut stoker sous forme d'un programme d'ordinateur PG1 des instructions participant à la mise en oeuvre des procédés décrits dans le présent document. Cette mémoire peut notamment stoker des instructions pour traiter des données ultrasonores et/ou des instructions pour construire des images illustratives du milieu observé.

Selon la configuration et le type de dispositif informatique considéré, la mémoire 13 peut être volatile (telle que la RAM), non volatile (telle que de la mémoire ROM, flash, EEPROM, etc. ou tout autre dispositif de stockage et/ou support lisible par ordinateur comme décrit ci-après) ou une combinaison des deux. La mémoire 13 peut, par exemple, être gérée en mode DMA (pour « Direct Memory Access » en anglais). La mémoire 13 utilisée par l'unité de traitement 11 peut par exemple comprendre tout ou partie d'une mémoire de carte graphique (ou carte vidéo), ce type de mémoire étant notamment apte à traiter et/ou envoyer des données d'image pouvant être utilisées pour afficher une ou des images sur un écran (ou une unité) d'affichage.

Plus généralement, le système 10 peut comprendre des dispositifs de stockage (amovibles et/ou non amovibles), y compris, mais sans s'y limiter, des disques magnétiques ou optiques ou des bandes.

En outre, le système 10 peut comporter un ou plusieurs dispositifs d'entrée tels qu'un clavier, une souris, un stylo, une entrée vocale, etc. et/ou un ou plusieurs dispositifs de sortie tels qu'un ou des écrans, des haut-parleurs, une imprimante, etc. L'environnement peut également comprendre une ou plusieurs connexions de communication, telles que des connexions de type LAN, WAN, point à point, etc. Dans certains modes de réalisation, les connexions peuvent être utilisées pour établir des communications point à point, des communications filaires, des communications sans fil, etc.

Le système 10 peut en outre comprendre au moins un support lisible par ordinateur, tel que la mémoire 13 notamment. Les supports lisibles par ordinateur peuvent être n'importe quel support disponible auquel l'unité de traitement 11 (notamment son ou ses processeurs) ou d'autres dispositifs comprenant l'environnement d'exploitation peuvent accéder. À titre d'exemple, et sans limitation, les supports lisibles par ordinateur peuvent comprendre des supports de stockage informatique et des supports de communication. Les supports de stockage informatique comprennent des supports volatils et non volatils, amovibles et non amovibles, mis en oeuvre dans tout procédé ou technologie de stockage d'informations telles que des instructions lisibles par ordinateur, des structures de données, des modules de programme ou d'autres données. Les supports de stockage informatique ne comprennent pas les supports de communication.

Le système 10 peut être un ordinateur unique fonctionnant dans un environnement en réseau utilisant des connexions logiques avec un ou plusieurs ordinateurs distants. L'ordinateur distant peut être une station de revue, un ordinateur personnel, un serveur, un routeur, un PC de réseau, un dispositif homologue ou un autre noeud de réseau commun, et peut comprendre généralement plusieurs ou tous les éléments décrits ci-dessus ainsi que d'autres non mentionnés. Les connexions logiques peuvent inclure toute méthode supportée par les supports de communication disponibles.

Comme illustré en figure 1, la sonde à ultrasons 20 peut comprendre par exemple un ou plusieurs transducteurs (dit aussi éléments transducteurs) notés 22, chacun étant configuré pour convertir un signal électrique SG1 reçu du système 10 en ondes ultrasonores (et éventuellement aussi réciproquement). Les transducteurs 22 peuvent ainsi être configurés pour émettre des ondes (ou impulsions ultrasonores, ou faisceaux ultrasonores) W1 dans le milieu M, ce qui correspond à une opération d'émission.

Les transducteurs 22 peuvent éventuellement aussi être configurés pour recevoir des signaux ultrasonores W2 du milieu M dans une opération de réception, par exemple en réponse aux ondes W1 transmises. Ces signaux W2 reçus peuvent prendre la forme d'un ensemble de signaux d'écho rétrodiffusés en réponse aux signaux W1 préalablement émis. Chaque transducteur 22 peut par exemple convertir un signal d'écho W2 reçu en un signal électrique. Le signal peut ensuite être traité par le système de pilotage 10 (par exemple par l'unité de traitement 11) ou par tout système associé (dédié), directement connecté ou non. Par exemple, le signal W2 peut être amplifié, filtré, numérisé et/ou une opération de conditionnement du signal peut être effectuée. Les éléments transducteurs peuvent être disposés selon une ligne de transducteurs, une matrice, ou comme un réseau de transducteurs ou toute autre configuration.

A noter que le ou les mêmes transducteurs 22 peuvent être utilisés pour émettre des ondes (ou impulsions) W1 et, le cas échéant, recevoir les ondes W2 formant la réponse du milieu M, ou des transducteurs différents peuvent être utilisés pour l'émission et la réception des ondes. Il peut y avoir un ou plusieurs transducteurs d'émission, et éventuellement une pluralité de transducteurs de réception. Dans une autre variante, un seul transducteur 22 peut être utilisé. Les transducteurs 22 peuvent comprendre des cristaux piézoélectriques et/ou d'autres composants qui peuvent être configurés pour générer et/ou enregistrer et/ou recevoir des signaux. Dans un souci de simplification de l'exposé, il est considéré dans les exemples de réalisation qui suivent que les transducteurs 22 sont des transducteurs piézoélectriques sans que cette précision ne soit en quoi que ce soit limitative.

Divers agencements du ou des transducteurs 22 sont possibles. Par exemple, un réseau de transducteurs 22 comprenant une pluralité d transducteurs peut être utilisé. Par exemple, on peut prévoir un réseau linéaire comprenant une pluralité (par exemple entre 2 et 10000) d'éléments transducteurs juxtaposés le long d'un axe X (direction horizontale ou direction du réseau X). Dans un exemple, la matrice est adaptée pour réaliser une imagerie bidimensionnelle (2D) du milieu M, mais la matrice pourrait également être une matrice bidimensionnelle adaptée pour réaliser une imagerie 3D du milieu M. En conséquence, une matrice de transducteurs 22 peut être utilisée. Il est cependant également possible que le système comprenne une seule ligne de transducteurs mobiles dans une sonde, de sorte que l'imagerie 3D puisse être réalisée. Il est également possible que le réseau de transducteurs comporte une ou des lignes d'éléments de transduction d'émission et une ou des lignes d'éléments de transduction de réception. Le réseau de transducteurs peut également être un réseau convexe comprenant une pluralité d'éléments transducteurs alignés le long d'une ligne courbe (par exemple dans une sonde courbe).

Selon un exemple, l'unité de traitement 11 est configurée pour recevoir des données du dispositif transducteur 20, traiter des données et/ou envoyer des données à un dispositif externe, comme par exemple, un dispositif de traitement, de stockage, d'affichage, un serveur, un ordinateur sur lequel un algorithme d'intelligence artificielle (IA) est exécuté, une station de travail dédiée, ou tout autre dispositif externe.

Le système 10 peut être un système d'imagerie, par exemple dans le domaine médical. Les images générées par le système peuvent être soit analysées en temps réel, par exemple par un utilisateur ou un algorithme, et/ou un module d'intelligence artificielle, soit analysées ultérieurement et/ou dans un autre lieu que celui dans lequel se trouve le système 10.

Le système 10 peut être un système médical, un système à ultrasons, voire un système médical à ultrasons. En conséquence, le dispositif 20 peut être une (ou faire partie, au moins partiellement, d'une) sonde médicale et/ou à ultrasons.

Par exemple, le système 10 peut être associé à une sonde à ultrasons 20, afin d'étudier un milieu M (figure 1), notamment pour collecter des données ultrasonores d'un tel milieu M. Le milieu M ainsi observé peut être de diverses natures selon le cas. Il peut s'agir par exemple graviers, de métaux, de tissus d'êtres vivants, en particulier des tissus humains ou tissus d'animaux. L'observation d'un milieu M comprenant une ou des structures minérales par exemple est également possible (graviers, volcan, cartographie d'un sol, par exemple de fonds marins, etc.).

Le système 10 peut ainsi être n'importe quel type de système électronique. Par exemple, le système 10 peut être un autre type de système médical qu'un système d'imagerie par ultrasons. En conséquence, le dispositif transducteur 20 peut être n'importe quel type de dispositif d'imagerie ou de capteur, utilisant d'autres ondes que les ondes ultrasonores (par exemple, des ondes ayant une longueur d'onde différente de la longueur d'onde des ultrasons et/ou des ondes n'étant pas des ondes sonores).

Selon d'autres exemples, le système 10 et/ou le dispositif transducteur 20 sont configurés à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine.

Des exemples de systèmes d'imagerie médicale comprennent un système d'imagerie par ultrasons, un système d'imagerie par rayons X (par exemple système permettant d'effectuer des mammographies) et un système IRM (Imagerie par résonance magnétique).

La figure 2 représente schématiquement des exemples de réalisation non limitatifs du système 10 tel que précédemment décrit en référence à la figure 1, à savoir un système d'imagerie ultrasonore dans les exemples considérés.

Le système d'imagerie ultrasonore 10 tel que représenté en figure 2 peut comprendre :
- une sonde à ultrasons 20 (correspondant par exemple au dispositif transducteur 20 de la figure 1),
- une unité de traitement 30 configurée pour traiter ou générer une image sur la base des signaux SG1 reçus par la sonde 20 (cette unité 30 correspondant par exemple à l'unité de traitement 11 de la figure 1),
- un panneau de commande 40 associé, lié ou connecté à l'unité de traitement 30, ledit panneau de commande pouvant comprendre au moins l'un parmi des boutons 41 et une tablette tactile 42, et
- un ou plusieurs écrans 50 configurés pour afficher la ou les images générées par l'unité de traitement 30.

La sonde 20 peut être connectée à l'unité de traitement 30 par tout moyen de connexion approprié tel qu'un câble 21 ou une connexion sans fil. La sonde 20 est en outre capable d'émettre des ondes ultrasonores W dans un milieu M, et éventuellement aussi de recevoir des ondes ultrasonores W du milieu M, lesdites ondes ultrasonores reçues pouvant alors être conséquentes ou résultant de réflexions desdites ondes ultrasonores émises sur des particules diffusantes à l'intérieur dudit milieu.

La sonde 20 peut être (ou comprendre) un réseau de transducteurs comprenant une pluralité de transducteurs (non représentés), chacun convertissant un signal électrique SG1 en une vibration et réciproquement. Un transducteur (dit aussi élément transducteur) est par exemple un élément piézoélectrique 22 tel que représenté en figure 1. Le réseau de transducteurs peut par exemple comprendre 128, 256 transducteurs ou d'avantage. Le réseau de transducteurs peut être linéaire ou incurvé et peut être disposé sur une surface extérieure du milieu M de manière à être couplé au milieu et à vibrer et à émettre ou recevoir des ondes ultrasonores W.

L'unité de traitement 30 peut comprendre des dispositifs récepteurs (non représentés), comprenant (ou étant) par exemple des circuits récepteurs, configurés pour traiter (par exemple amplifier et/ou filtrer) les signaux SG1 reçus de la sonde 20. Ces dispositifs récepteurs peuvent par exemple comprendre des convertisseurs pour transformer les signaux reçus en données représentant le signal (par exemple des convertisseurs analogiques-numériques (ADC) configurés pour transformer une tension en un code numérique). Les données obtenues peuvent être traitées de diverses manières ; elles peuvent notamment être stockées temporairement dans une mémoire accessible à l'unité de traitement ou traitées directement pour calculer des données traitées intermédiaires (données formées en faisceau, dites « beamformed data » en anglais). L'unité de traitement 30 peut mettre en oeuvre toute méthode de traitement connue pour générer et/ou traiter une ou plusieurs images ou cartes ou données sur la base des signaux reçus de la sonde, telle que la formation de faisceaux.

Les données traitées peuvent être associées à une image ultrasonore de différents natures, lesquelles peuvent être :
- une image B-mode du milieu (image en mode B) généralement affichée en niveaux de gris permettant de visualiser les organes à l'intérieur du milieu, et/ou
- une image dite Doppler, montrant les mouvements de fluides dans le milieu observé, par exemple la vitesse de mouvement et/ou l'écoulement de fluides dans le milieu (image utilisant souvent des codes couleurs), par exemple utile pour visualiser les vaisseaux sanguins et leurs activités dans le milieu, ou
- une image montrant une caractéristique mécanique du milieu (élasticité), par exemple utile pour identifier des zones de dureté différente qui peuvent se révéler être des tumeurs présentes à l'intérieur du milieu (par exemple des données d'image d'élastographie par ondes de cisaillement (ShearWave^{™} Elastography)).

L'écran d'affichage 50 (par exemple monté sur un bras de support 51) peut être un écran de visualisation de l'image traitée par l'unité de traitement 30 et/ou peut afficher diverses informations selon le cas d'usage, notamment des informations d'aide ou une aide gestuelle contextuelle adaptée ou adaptable au moyen du pavé tactile 42.

Le panneau de commande 40a constitue tout ou partie d'une l'interface utilisateur utilisable par un utilisateur pour interagir (commander, recevoir des informations, etc.) avec le système 10.

Un premier exemple de réalisation du système 10, noté ci-après 90, est à présent décrit en référence à la figure 3. Ce système 90 ne met pas oeuvre le concept de la présente divulgation.

Plus précisément, le système de pilotage 90 comprend au moins un pulseur 101 configuré pour piloter un transducteur 22 du dispositif 20 émetteur d'ondes. A cet effet, le pulseur 101 est configuré pour générer et envoyer des signaux de sorties SG1 au transducteur 22. Ainsi, ces signaux électriques SG1 sont émis depuis la sortie OUT1 du pulseur vers l'entrée IN2 du dispositif émetteur 20, causant l'émission d'ondes ultrasonores W1 par le transducteur 22 dans en direction du milieu M, comme déjà décrit. Bien que le système 10 puisse comprendre une pluralité de pulseurs 101, on considère par souci de simplification de l'exposé qu'un seul pulseur 101 est mis en oeuvre dans cet exemple.

Comme illustré en figure 3, l'unité de commande 12 comprend des régulateurs de tension 102a et 102b connectés respectivement aux bornes d'alimentation positive SHV+ et négative SHV- du pulseur 101. De façon connue, ces régulateurs 102a et 102b (dit aussi PSU pour « Power Supply Unit » en anglais) sont configurés pour délivrer une tension statique (ou tension constante) aux bornes d'alimentation SHV+/SHV- du pulseur 101. Ces régulateurs peuvent être programmables pour fixer une tension d'alimentation pour un mode de fonctionnement ou une sonde 20 donné. Ces régulateurs 102a, 102b fournissent ainsi une puissance au pulseur 101 qui est fixe dans le temps. En pratique, de tels régulateurs de tension peuvent être implémentés par exemple dans une carte d'alimentation de l'unité de commande 12.

L'arrangement du système 90 de la figure 3 présente cependant les problèmes et déficiences tels que précédemment décrits (défaillances, manque de fiabilités, performances non satisfaisantes, etc.), en raison notamment d'une mauvaise maîtrise des signaux de sortie SG1 émis par le pulseur 101 vers le dispositif émetteur 20.

Des exemples de réalisation du système de pilotage 10 (figure 1), noté par la suite 100, qui mettent en oeuvre le concept de la présente divulgation, vont à présent être décrits ci-après en référence aux figures 4-15. Sauf indication contraire, les éléments décrits ci-avant en référence au système de pilotage 10 s'applique de façon analogue au système de pilotage 100. Comme déjà indiqué, on suppose dans ce qui suit que le système 100 est un système d'imagerie ultrasonore bien que d'autres implémentations soient possibles. Par souci de simplification de l'exposé, certains éléments décrits ci-avant en référence aux figures 1-3 ne sont pas à nouveau décrits en détail ci-après.

Selon un exemple, le système de pilotage 100 est positionné à l'arrière des connecteurs de sonde, par exemple derrière les connecteurs 32 représentés en figure 2. Selon un exemple, le système de pilotage 100 est positionné à l'interface entre le câble 21 et la sonde 20, ou dans la sonde 20 elle-même.

Plus précisément, la figure 4 représente schématiquement selon un exemple le système de pilotage (ou système de commande) 100 comprenant une unité de commande 12 configuré pour piloter un dispositif émetteur 20, à savoir un dispositif transducteur ultrasonore dans les exemples qui suivent. Le système de pilotage 100 et le dispositif transducteur 20 forment ensemble un système noté SY1.

Comme déjà indiqué, l'unité de commande 12 comprend un ou des pulseurs 101 configurés pour piloter le dispositif transducteur 20 au moyen de signaux électrique SG1. Les signaux électriques SG1 générés par le ou les pulseurs 101 sont envoyés au dispositif transducteur 20 pour causer l'émission d'ondes ultrasonores W1 dans le milieu M. Les signaux électriques SG1 ainsi générés par le pulseur 101 sont représentatifs des ondes ultrasonores W1 envoyées pour exciter le milieu M.

Le dispositif transducteur 20 peut comprendre un ou des transducteurs ultrasonores 22 (par exemple de type piézoélectriques), chaque transducteur étant piloté par les signaux SG1 d'un pulseur respectif 101 du système 100.

Comme illustré en figure 4, chaque pulseur 101 peut utiliser une liaison électrique pour échanger des signaux électriques SG1 avec le dispositif transducteur 20, en émission et éventuellement aussi en réception. A titre d'exemple, le système 100 peut comprendre 256 pulseurs 101 configurés pour piloter des transducteurs 22 respectifs du dispositif transducteur 20.

Les liaisons électriques utilisés pour transmettre les signaux de sortie SG1 au dispositif transducteur 20 peuvent être (ou comprendre) des liaisons (ou connexions) physiques électriquement conductrices dont l'agencement peut varier selon le cas. Chaque liaison LN peut être formée par exemple en tout ou partie par au moins une piste électrique, par exemple une ou des pistes électriques formées sur une carte électronique (par exemple la carte de pilotage 32 illustrée en figure 2). Chaque liaison peut éventuellement comprendre au moins une portion d'un câble ou un câble électrique (par exemple de type coaxial), tel que le câble 21 illustré en figure 2. Selon un exemple, une telle liaison électrique peut comprendre au moins une portion de piste électrique, et éventuellement aussi au moins une portion de câble électrique.

Sauf indication contraire, on considère dans ce qui suit par souci de simplification de l'exposé de la divulgation que le système de pilotage 100 utilise un unique pulseur 101 pour piloter un transducteur 22 du dispositif transducteur 20, bien que des variantes soient possibles où une pluralité de pulseurs 101 sont utilisés pour piloter un ou des transducteurs 22 du dispositif 20. Les modes de réalisation de la présente divulgation s'appliquent de façon analogue au pilotage d'un ou de transducteurs 22 par une pluralité de pulseurs 101.

Selon un exemple, l'unité de commande 12 est en outre apte à recevoir des signaux électriques SG1 en provenance du dispositif transducteur 20 suite à excitation induite par les ondes W1 émises. Ces signaux SG1 sont représentatifs d'ondes W2 (figure 1) reçues par le dispositif transducteur 20 en provenance du milieu M. Ces ondes W2 forment par exemple un écho ultrasonore, c'est-à-dire une réponse du milieu M aux ondes ultrasonores W1.

Le pulseur 101 (et plus généralement l'unité de commande 12) est contrôlé par l'unité de traitement 11. Cette dernière peut en particulier commander le pulseur 101 pour générer des signaux de sorties SG1 appropriés selon l'application considérée. Comme illustré en figure 1, l'unité de traitement 11 peut comprendre au moins un processeur et une mémoire non volatile 13. Le système de pilotage 10 est ainsi configuré pour mettre en oeuvre un procédé de pilotage pour piloter le dispositif transducteur 20 comme décrit ci-après dans des modes de réalisation particuliers. À cet effet, le système 10 peut comprendre un programme d'ordinateur PG1 stocké dans la mémoire non volatile 13, ce programme d'ordinateur PG1 comprenant tout ou partie des instructions pour la mise en oeuvre dudit procédé. Le processeur est configuré pour exécuter notamment les instructions définies par le programme d'ordinateur PG1.

La mémoire non volatile 13 (figure 1) peut correspondre à un quelconque dispositif de stockage (amovible et/ou non amovible) tel que celui décrit ci-avant en référence au système 10. Le dispositif de pilotage 60 peut également comprendre au moins un support lisible par ordinateur comme précédemment décrit en référence au système 10 (par exemple des supports de stockage informatique et/ou des supports de communication).

De façon analogue au système 90 (figure 3), le système de pilotage 100 représenté en figure 4 comprend des régulateurs de tension 102a et 102b configurés pour alimenter électriquement le pulseur 101 à ses bornes d'alimentation, à savoir ses bornes d'alimentation positive SHV+ et SHV-respectivement. Pour ce faire, les régulateurs 102a et 102b (dit aussi PSU) délivrent une tension statique ou tension constante (dit aussi rail d'alimentation) pour alimenter le pulseur 101 à ses bornes d'alimentation.

Le système de pilotage 100 (figure 4) diffère cependant du système 90 (figure 3) en ce qu'il comprend en outre au moins un contrôleur 120, dit aussi bloc (ou unité) de modulation, configuré pour moduler les signaux de sortie SG1 émis depuis le pulseur 101 pour piloter le transducteur 22. Autrement dit, le ou les contrôleurs 120 sont agencés dans le système 100 (à savoir dans l'unité de commande 12 et/ou dans le dispositif transducteur 20 dans les exemples considérés) pour réaliser une modulation F1 des signaux de sortie SG1 générés en sortie OUT1 du pulseur 101 et envoyés en entrée IN2 du transducteur 22.

Le nombre de contrôleurs 120 ainsi que leur implémentation peuvent être adaptés selon le cas. Quelle que soit la forme du contrôleur 120 considéré, celui-ci a pour fonction de moduler (ou contrôler dans le temps) les signaux de sortie SG1 envoyés par le pulseur 101 au dispositif transducteur 20. Le système 100 peut ainsi inclure un unique contrôleur 120 ou une pluralité de contrôleurs 120 pour effectuer la modulation F1.

Dans la présente divulgation, la notion de modulation couvre toutes modifications au cours du temps d'au moins une caractéristique d'un signal, telle que sa puissance, sa tension, son courant, son spectre fréquentielle, son temps de montée (« rise time » en anglais) et/ou temps de descente (« fall time » en anglais), etc.

La modulation sur une période donnée d'un signal, tel que les signaux de sortie SG1 émis depuis le pulseur 101, implique cependant que ledit signal n'est pas nul sur la période considérée. Autrement dit, la modulation au sens de la présente divulgation exclut le cas où ledit signal modulé serait nul (fixé à zéro). La modulation au sens de la présente divulgation vise à modifier un signal non nul au cours du temps.

Des exemples d'implémentation du ou des contrôleurs 120 sont à présent décrits ci-après en référence à la figure 4. En particulier, le système 100 peut comprendre l'un quelconque, ou une pluralité, parmi les contrôleurs 120 suivants (figure 4) :
- un contrôleur d'alimentation 122a et/ou 122b ;
- un contrôleur de sortie 124 ; et
- un contrôleur de retour 126.

Selon un exemple, le système de pilotage 100 comprend les contrôleurs d'alimentation 122a et/ou 122b.

Selon un exemple, le système de pilotage 100 comprend le contrôleur de sortie 124.

Selon un exemple, le système de pilotage 100 comprend le contrôleur de retour 126.

Selon un exemple, le système de pilotage 100 comprend les contrôleurs d'alimentation 122a et/ou 122b, ainsi que le contrôleur de retour 126.

Selon un exemple, le système de pilotage 100 comprend les contrôleurs d'alimentation 122a et/ou 122b, le contrôleur de sortie 124 et le contrôleur de retour 126.

Plus particulièrement, selon un exemple, le contrôleur 120 comprend un (ou au moins un) contrôleur d'alimentation, 122a et/ou 122b, connecté à une borne d'alimentation du pulseur, à savoir à la borne d'alimentation positive SHV+ et/ou négative SHV- dans le cas présent (figure 4). Le système 100 peut ainsi comprendre uniquement le contrôleur 122a d'alimentation positive, ou uniquement le contrôleur 122b d'alimentation négative, ou les deux. Les contrôleurs d'alimentation 122a et 122b sont configurés pour contrôler respectivement dans le temps (ou moduler) une tension d'alimentation V+ et V-du pulseur 101.

L'installation d'un contrôleur d'alimentation 122a/122b implique avantageusement une mise en oeuvre de complexité limitée, et donc de mise en oeuvre aisée, pour moduler les signaux de sortie, notamment car le contrôleur d'alimentation est référencé à une tension fixe positive ou négative (celle délivrée par les régulateurs 102a/102b), et non à une tension variable bipolaire comme c'est le cas pour le contrôleur de sortie 124 par exemple qui reçoit la tension délivrée en sortie par le pulseur 101.

Par leur position aux bornes d'alimentation du pulseur 101, les contrôleurs d'alimentation 122a et 122b sont particulièrement efficaces pour contrôler les signaux délivrés par les régulateurs 102a et 102b, respectivement. Cela permet notamment de protéger les régulateurs 102a et 102b, par exemple en cas de court-circuit ou surintensité en aval des contrôleurs d'alimentation 122a et 122b.

Il est possible de n'utiliser qu'un seul des deux contrôleurs d'alimentation, à savoir soit 122a soit 122b, par exemple si l'on souhaite contrôler la symétrie du signal de sortie SG1 du pulseur 101. On peut par exemple moduler la partie positive (ou négative, respectivement) du signal de sortie SG1 en fonction de ce qui a été généré préalablement par la partie négative (ou positive, respectivement) dudit signal. Ainsi, on peut par exemple obtenir un signal de sortie SG1 généré par un sous-circuit négatif (ou positif) du pulseur 101 pendant une première période, puis adapter la signal de sortie SG1 pendant une deuxième période (ultérieure à la première période) pour le rendre symétrique et/ou compenser (ou adapter) certaines caractéristiques de signal obtenues pendant la première période.

Selon un exemple, le pulseur 101 comprend une borne de masse GND1 qui est considérée comme une borne d'alimentation et qui peut être connectée à un contrôleur d'alimentation analogue aux contrôleurs 122a et 122b pour contrôler dans le temps une tension d'alimentation à cette borne de masse.

De façon générale, un contrôleur d'alimentation au sens de la présente divulgation peut donc être connecté est l'une quelconque, ou une pluralité, parmi : la borne d'alimentation positive SHV+, la borne d'alimentation négative SHV- et la borne de masse GND1 du pulseur 101.

Selon un exemple, le contrôleur d'alimentation 122a connecté à la borne d'alimentation SHV+ est configuré pour moduler une puissance délivrée au pulseur 101 sur sa borne d'alimentation SHV+ à partir d'une tension statique délivrée par le régulateur de tension 102a. De même, selon un exemple, le contrôleur d'alimentation 122b connecté à la borne d'alimentation SHV- est configuré pour moduler une puissance délivrée au pulseur 101 sur sa borne d'alimentation SHV- à partir d'une tension statique délivrée par le régulateur de tension 102b.

Comme déjà indiqué en référence au système 90 (figure 3), les régulateurs 102a et 102b du système 100 (figure 4) peuvent être programmés ou configurés pour délivrer une tension statique (ou constante) au cours du temps, cette tension étant par exemple fixée en fonction du mode de fonctionnement ou de la sonde 20 utilisée. Dans cet exemple, les contrôleurs d'alimentation 122a et/ou 122b, alimentés en tension par les régulateurs 102a et/ou 102b respectivement, sont configurés pour modifier au cours du temps la puissance délivrée au pulseur 101.

Selon un exemple, le contrôleur 120 comprend un (ou est un) contrôleur de sortie 124 connecté en sortie du pulseur 101, c'est-à-dire sur la borne de sortie OUT1 destinée à émettre en sortie les signaux de sortie SG1 pour piloter le transducteur 22 du dispositif transducteur 20. Le contrôleur de sortie 124 est alors configuré pour contrôler dans le temps (ou moduler) les signaux de sortie SG1 envoyés au dispositif transducteur 20 pour générer les ondes ultrasonores W1 vers le milieu M.

L'installation d'un contrôleur de sortie 124 permet avantageusement de limiter le courant de sortie et/ou la tension de sortie du pulseur 101, afin notamment de ne pas stresser ou endommager le pulseur 101 et/ou le transducteur 22, dans la mesure où le pulseur 101 pilote le transducteur 22. Le contrôleur de sortie 124 étant positionné entre le pulseur 101 et le transducteur 22, et donc au plus proche du transducteur 22, il est en particulier capable de contrôler efficacement les signaux de sortie SG1 émis par le pulseur 101 et envoyés au transducteur 22.

Selon un exemple, le contrôleur de sortie 124 est configuré pour moduler au moins l'une des caractéristiques électriques des signaux de sortie SG1 du pulseur 101 parmi la tension (V), le courant (I) et la puissance (P) desdits signaux de sortie SG1.

Selon un exemple, le contrôleur 120 comprend un (ou est un) contrôleur de retour 126 connecté sur une borne de retour OUT2 du transducteur 22 du dispositif 20. Le contrôleur de retour 126 est alors configuré pour contrôler dans le temps (ou moduler) des signaux de retour SG2 du transducteur 22.

Plus précisément, chaque transducteur 22 du dispositif transducteur 20 peut par exemple former un dipôle électrique comprenant une borne d'entrée IN2 pour recevoir les signaux de sortie SG1 d'un pulseur 101 et une borne de retour (ou borne de sortie) OUT2 connectée à une masse de référence (dite aussi « return path » en anglais). Cette borne de retour OUT2 du transducteur 22 peut par exemple être connectée en série à une masse du dispositif transducteur 20 ou à toute autre masse de référence du système de pilotage 100 (ou plus généralement du système SY1), voire connectée en série à un autre composant électrique (par exemple un autre pulseur) du système de pilotage 100.

En installant un contrôleur de retour 126 connecté en série à la borne de retour OUT2 et à la masse de référence, on peut ainsi moduler la puissance des signaux de sortie SG1 émis depuis le pulseur 101 pour piloter le transducteur 22.

L'installation du contrôleur de retour 126 implique avantageusement une mise en oeuvre de complexité limitée, et donc de mise en oeuvre aisée, pour moduler les signaux en sortie du transducteur 22, et donc par conséquent les signaux de sortie SG1 du pulseur 101. Dans la mesure où le contrôleur de retour 126 est connecté en série avec la borne de retour OUT2, et donc proche du transducteur 22, le contrôleur de retour 126 est particulièrement efficace pour contrôler les signaux en sortie du transducteur 22 et donc pour protéger le transducteur 22.

Le système 100 peut comprendre un seul parmi les contrôleurs 122-126 ou une quelconque combinaison parmi ces contrôleurs 120 (par exemples les contrôleurs 122a, 122b et 124). Par souci de simplification de l'exposé, on suppose par la suite que le système SY1 comprend des contrôleurs 120, par exemples les contrôleurs 122a, 122b, 124 et 126 tels que précédemment décrits, pour moduler en puissance les signaux de sortie SG1 du pulseur 101 destinés à piloter le transducteur 22.

Il est possible de n'utiliser qu'un seul contrôleur parmi les contrôleurs 122a, 122b, 124 et 126 précédemment décrits, par exemple uniquement le contrôleur 122a, uniquement le contrôleur 122b ou uniquement le contrôleur 126. Les contrôleurs d'alimentation 122a, 122b permettent avantageusement de limiter le courant et/ou la tension d'alimentation du pulseur, notamment pour ne pas stresser/endommager en particulier le pulseur 101. Le contrôleur de sortie 124 et/ou le contrôleur de retour 126 permettent avantageusement de limiter le courant et/ou la tension appliqués au transducteur 22, notamment pour ne pas stresser/endommager en particulier le transducteur 22.

Diverses implémentations de ces contrôleurs 120 sont possibles pour permettre la modulation F1. A titre d'exemple, le ou les contrôleurs 120 peuvent être mis en oeuvre sous la forme d'un ou plusieurs composants discrets et/ou intégrés, par exemple un circuit intégré. Le recours à des composants discrets permet notamment un montage robuste et dense, par exemple dans une carte électronique.

Selon un exemple, les contrôleurs 120 prennent la forme d'un circuit intégré, par exemple de type FPGA (« Field-programmable gate array » en anglais).

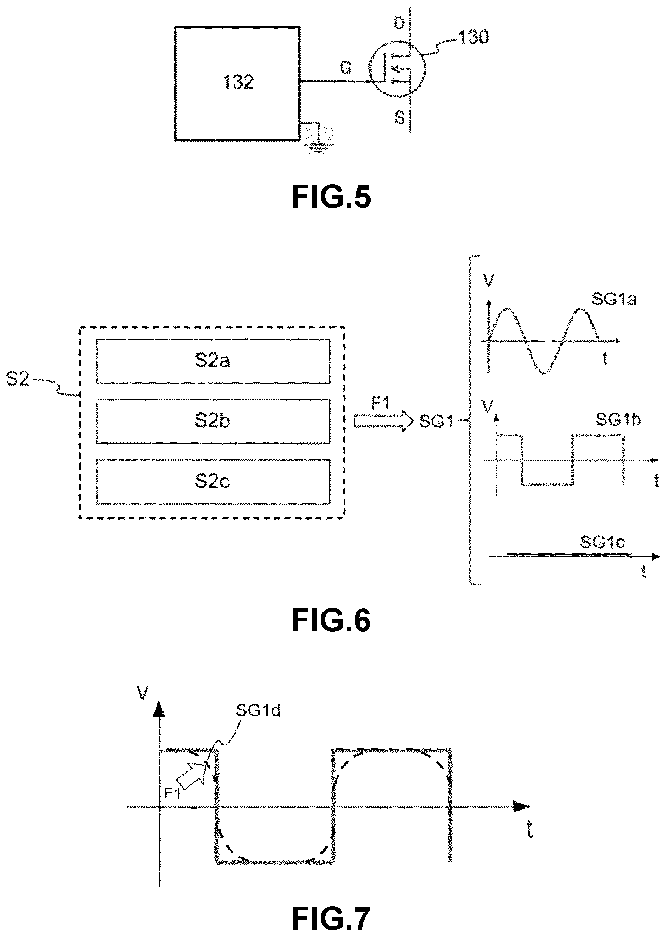

La figure 5 illustre schématiquement un exemple de mise en oeuvre d'un contrôleur 120 au sens de la présente divulgation. Comme représenté, le contrôleur 120 prend dans cet exemple la forme d'un transistor MOSFET (transistor à effet de champ à grille isolée) dont la tension de grille est modulée au cours du temps, par exemple au moyen d'une unité de commande comprenant un convertisseur CNA (numérique vers analogique ou DAC pour « Digital to Analog ») noté 132.

Pour ce faire, le convertisseur 132 est configuré pour envoyer des commandes sur la grille G du MOSFET. Dans le cas d'un contrôleur d'alimentation 122a/122b, l'une parmi les bornes de drain (D) et de source (S) peut être connectée au régulateur 102a/102b respectivement et l'autre à la borne d'alimentation correspondante SHV+/SHV- du pulseur 101. De même, dans le cas du contrôleur de sortie 124, l'une parmi les bornes de drain (D) et de source (S) peut être connectée à la borne de sortie OUT1 du pulseur 101 et l'autre en série avec l'entrée IN2 du transducteur 22.

L'exemple de la figure 5 permet avantageusement de mettre en oeuvre les contrôleurs 120 avec un niveau de complexité limité et un bas coût d'implémentation. Il est ainsi possible de faciliter la mise au point (conception), la mise en oeuvre et la maintenance du dispositif émetteur d'ondes. Des implémentations plus complexes sont toutefois possibles.

Un procédé de pilotage mis en oeuvre par le système de pilotage 100 tel que précédemment décrit (figures 4-5) est à présent décrit conjointement aux figures 6-9 selon des modes de réalisation particuliers. A cet effet, le système de pilotage 100 (ou plus généralement le système SY1) peut exécuter le programme d'ordinateur PG1. Le système 100 peut exécuter des instructions d'au moins un programme d'ordinateur, dont par exemple le programme PG1.

Au cours d'une étape S2 de modulation, les signaux de sortie SG1 du pulseur 101 sont modulés par un (ou au moins un) contrôleur 120. Cette modulation - notée F1 - peut être réalisée par un contrôleur 120 selon au moins l'un parmi :
a) un contrôle (S2a) d'une tension d'alimentation (V+ et/ou V-) du pulseur 101 par un contrôleur d'alimentation 122a et/ou 122b connecté à une borne d'alimentation IHV+ et/ou IHV- du pulseur 101 ;
b) un contrôle (S2b) de signaux de sortie SG1 par un contrôleur de sortie 124 connecté en sortie OUT1 du pulseur 101 ; et
c) un contrôle (S2c) de signaux de retour SG2 du transducteur 22 par un contrôleur de retour 126 connecté sur une borne de retour OUT2 du transducteur 22.

Autrement dit, la modulation F1 est réalisée au moyen de l'un quelconque, ou de plusieurs, parmi les contrôleurs 120 précédemment décrits. Cette modulation F1 permet un contrôle dans le temps des signaux de sortie SG1 émis depuis le pulseur 101 pour piloter le transducteur 22. En particulier, la puissance et/ou la forme des signaux SG1 envoyés en entrée IN2 du transducteur 22 peuvent être adaptées ou modifiées au cours du temps.

Selon un exemple, la modulation F1 cause une variation au cours du temps d'au moins une caractéristique des signaux de sortie SG1, cette caractéristique prenant au moins deux valeurs non nulles distinctes au cours de ladite modulation.

Selon un exemple, les contrôleurs d'alimentation 122a et/ou 122b sont utilisés pour réaliser la modulation F1 des signaux de sortie SG1.

Selon un exemple, le contrôleur de retour 126 est utilisé pour réaliser la modulation F1 des signaux de sortie SG1.

Selon un exemple, les contrôleurs d'alimentation 122a et/ou 122b sont utilisés en combinaison avec le contrôleur de retour 126 pour réaliser la modulation F1 des signaux de sortie SG1.

Selon un exemple, la forme et/ou l'amplitude de la tension transmise au transducteur 22 sont modulées (ou adaptées). En conséquence, pour une impédance donnée du transducteur 22 (ou plus généralement de la sonde), le courant et la puissance transmis sont par conséquent modifiés.

La modulation F1 permet avantageusement d'améliorer le pilotage du dispositif transducteur 20 et donc les performances et la fiabilité du système SY1 dans son ensemble. La modulation F1 permet en particulier de contrôler efficacement et de façon flexible les signaux de sortie SG1 envoyés au transducteur 22 et ainsi de s'assurer que les valeurs limites théoriques des paramètres (notamment en courant, tension et/ou puissance) du transducteur 22, et plus généralement de la chaîne de transmission depuis le pulseur 101 au transducteur 22, sont respectées. En maîtrisant plus précisément le pilotage du transducteur 22, on peut éviter de stresser excessivement le système, limiter les défaillances (pannes, dégradations, etc.), assurer une bonne fiabilité du système et garantir que le système présente des performances satisfaisantes.

Grâce à la modulation F1, il est notamment possible d'adapter les ondes envoyées avec plus de flexibilité (en puissance, fréquence, durée, etc.) sans détériorer le pulseur 101 ou le transducteur 22, ce qui permet de s'affranchir de certains compromis ou limitations (notamment en termes de puissance) avec lesquels il faut habituellement composer avec ce type de dispositif pour garantir de bonnes performances et un vieillissement normal de l'ensemble. Contrairement aux systèmes classiques, il est possible grâce au concept de la présente divulgation de configurer le dispositif transducteur 22 de sorte à émettre les ondes désirées, c'est-à-dire conformes aux caractéristiques d'ondes souhaitées selon l'application visée.

Avec une modification limitée de l'ensemble, et donc un faible surcoût, on peut ainsi avantageusement obtenir un dispositif transducteur 22 présentant des capacités de haut niveau.

En améliorant le pilotage du transducteur 22, on peut par exemple améliorer la qualité d'image, telle que par exemple la qualité d'une image ultrasonore, dans une application d'imagerie ultrasonore, par exemple dans le domaine médical. Grâce au procédé de la présente divulgation, il est notamment possible d'obtenir une bonne qualité d'image.

Diverses implémentations de la modulation F1 des signaux de sortie SG1 sont possibles. Comme illustré en figure 6, la modulation F1 est par exemple configurée pour contrôler (ou adapter) la forme d'onde des signaux de sortie SG1. La modulation F1 est par exemple configurée pour causer une modification de la forme d'onde des signaux de sortie SG1 par rapport à la forme d'onde générée intrinsèquement en sortie du pulseur 101 (c'est-à-dire sans modulation F1). Il est ainsi possible d'adapter la forme d'onde des signaux SG1 (et donc des ondes W1) pour un pulseur 101 donné, et ainsi rendre le pilotage du transducteur 22 plus flexible car moins dépendant du type de pulseur 101 utilisé. On peut notamment adapter la forme des signaux SG1 aux contraintes et spécificités des différents modes d'imagerie susceptibles d'être utilisés (par exemple B-mode, mode Doppler, ShearWave^{™} Elastography, etc.).

A titre d'exemple, la modulation F1 peut causer la génération, en sortie du pulseur 101, de signaux de sortie SG1 de forme sinusoïdale (signal SG1a, figure 6), de forme carrée, de forme intermédiaire ou hybride (signal SG1d, figure 7), ou de tout autre forme appropriée selon le cas d'usage. En variante, la modulation F1 peut causer la désactivation du pulseur 101 ce qui revient à générer un signal de sortie SG1 nul (signal SG1c, figure 6).

Plus précisément, comme illustré en figure 7, on suppose par exemple que le pulseur 101 est de type « pulsé », à savoir un pulseur configuré intrinsèquement pour générer des signaux de sortie SG1 de forme carrée. Selon un exemple, la modulation F1 réalisée au moyen du ou des contrôleurs 120 cause la modification de la forme d'onde depuis la forme intrinsèque carrée dans une forme d'onde modifiée (ou forme d'onde modulée), à savoir une forme d'onde sinusoïdale ou une forme intermédiaire (ou hybride) SG1d entre la forme carrée et la forme sinusoïdale. En rapprochant la forme d'onde de la forme sinusoïdale, on peut avantageusement limiter les harmoniques présents dans les signaux SG1 transmis au transducteur 22, ce qui permet avantageusement de limiter la dissipation de chaleur et les rayonnements électromagnétiques, d'améliorer le rendement énergétique du système et de prolonger la durée de vie du système.

Selon un exemple, la modulation F1 est réalisée au moyen au moins des contrôleurs d'alimentation 122a et/ou 122b. La modulation F1 cause une variation au cours du temps des tension d'alimentation respectives V+ et/ou V-du pulseur 101, ces tensions variant selon (ou prenant) au moins deux valeurs non nulles distinctes au cours de ladite modulation. Ainsi, les tensions d'alimentation V+ et/ou V- varient selon une pluralité de valeurs de tension distinctes et non nulles tandis que le pulseur 101 génère en sortie des signaux de sortie SG1 non nulles. Cela permet avantageusement de créer des signaux de sortie SG1 de formes variées, ce qui offre plus de flexibilité et un meilleur contrôle du pulseur 101. Avec un même pulseur 101, on peut avantageusement adapter la forme d'onde des signaux de sortie SG1 par modulation F1. A partir d'un pulseur 101 donné de type « pulsé » (configuré intrinsèquement pour délivrer des signaux de sortie SG1 carrés), on peut avantageusement causer par modulation F1 la génération en sortie du pulseur 101 de signaux de sortie SG1 de forme sinusoïdale, ou de forme d'onde intermédiaire entre carrée et sinusoïdale. Autrement dit, en appliquant la modulation F1 aux tensions d'alimentation V+ et/ou V- du pulseur 101, on peut avantageusement transformer un pulseur 101 de type « pulsé » en un pulseur 101 de type linéaire, c'est-à-dire apte à délivrer des signaux de sortie SG1 de formes variées, notamment de forme sinusoïdale ou intermédiaire.

Selon un exemple particulier, la modulation F1 est configurée pour contrôler (ou adapter) la puissance des signaux de sortie SG1 émis depuis le pulseur 101 pour piloter le transducteur 22.

Selon un exemple, la modulation F1 est réalisée à une fréquence de modulation Fm supérieure ou égale à une fréquence de tir Ft à laquelle le transducteur 22 émet des ondes W1 en réponse aux signaux de sortie SG1 du pulseur 22. La fréquence de modulation Fm peut par exemple être de l'ordre de la fréquence de tir Ft.

A titre d'exemple, la fréquence de tir Ft est comprise entre 100 kHz et 20 MHz (voire entre 1 MHz et 20 MHz pour l'émission d'ondes W1 acoustiques, par exemple dans des applications d'imagerie médicale). Dans ce cas, la fréquence de modulation Fm peut être telle que Fm ≥ 100 kHz (où 100 kHz est la limite basse de la fréquence de tir).

Selon un exemple, la modulation F1 est réalisée à une fréquence de modulation Fm supérieure ou égale à une fraction 1/P de la fréquence de tir Ft, où P est un entier égal à 10 par exemple. La fréquence de modulation Fm peut selon certains exemples être inférieure à la fréquence de tir Ft. Dans un cas par exemple où une séquence de tir de quelques périodes d'un signal carré est réalisée, cette séquence peut avantageusement être modulée selon une enveloppe gaussienne (amplitude faible au début du tir, maximale au milieu et de nouveau faible à la fin). Dans ce cas, la fréquence Fm est inférieure à la fréquence de tir Ft, mais doit être supérieure à la fréquence maximale de variation de la tension d'alimentation délivrée par les régulateurs 102a et 102b.

Selon un exemple, les signaux de sortie SG1 sont modulés dans une bande fréquentielle notée Bm. Le transducteur 22 (ou plus généralement le dispositif transducteur 20) est caractérisé par une bande passante dans laquelle il est apte à convertir des signaux de sortie SG1 en des ondes ultrasonores W1. La bande fréquentielle Bm est alors configurée pour que son intersection avec ladite bande passante soit non nulle.

Le système de pilotage 100, et plus généralement le système SY1, peuvent par exemple être mis en oeuvre dans des applications d'imagerie médicale, notamment appliqués aux êtres vivants (humains et/ou animaux).

Selon un exemple, la modulation F1 des signaux de sortie SG1 est réalisée en continu, ou de façon discrète, au cours du temps.

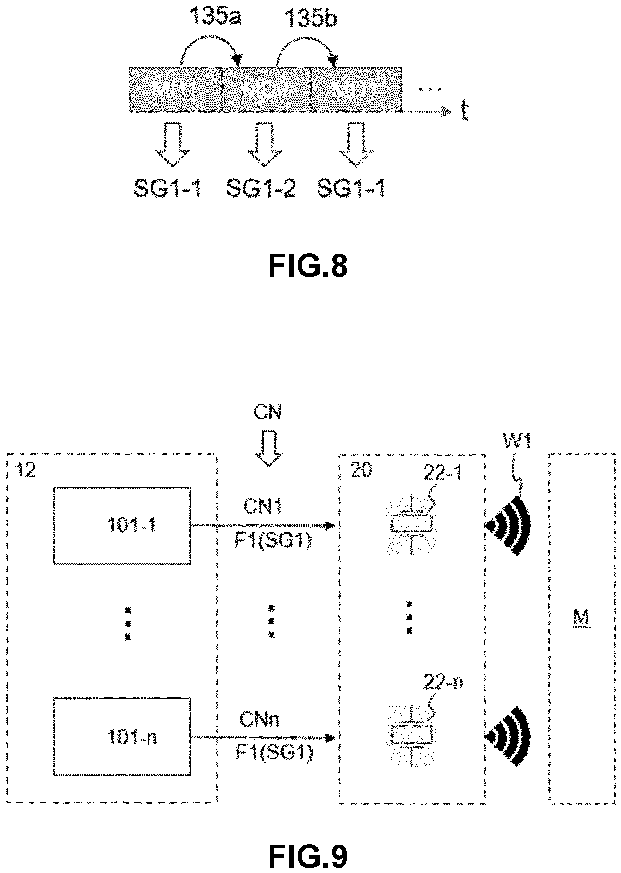

Comme illustré en figure 8 selon un exemple, la modulation F1 réalisée au moyen du ou des contrôleurs 120 peut comprendre un basculement 135a entre deux modes, à savoir :
- un premier mode MD1 causant l'émission par le pulseur 101 de signaux de sortie SG1 (notés SG1-1) selon une première configuration de forme d'onde et/ou de puissance ; et
- un deuxième mode MD2 causant l'émission par le pulseur 101 de signaux de sortie SG1 (notés SG1-2) selon une deuxième configuration de forme d'onde et/ou de puissance, différente de la première configuration.

En adaptant les modulations réalisées dans les modes MD1 et MD2, on peut ainsi contrôler de façon flexible le contrôle du transducteur 22 et donc assurer des bonnes performances et un bonne fiabilité du système. A titre d'exemple, on peut configurer le ou les contrôleurs 120 de sorte que :
- dans le premier mode MD1, aucune modulation F1 n'est réalisée (désactivation du ou des contrôleurs 120) ; les signaux de sortie SG1-1 sont les signaux générés intrinsèquement par le pulseur 101 en l'absence de modulation ; et
- dans le deuxième mode MD2, une modulation F1 est réalisée (activation du ou des contrôleurs 120) de façon à ce que les signaux de sortie SG1-2 ainsi modulés soient différents de ceux émis dans le premier mode MD1.

Selon un exemple, dans le deuxième mode MD2, les signaux de sortie SG1-2 sont bridés, c'est-à-dire limités en puissance par rapport aux signaux de sortie intrinsèques, de sorte que le transducteur 22 opère en mode dégradé (émission d'ondes W1 moins puissante qu'en mode MD1 sans modulation).

On peut ainsi configurer le ou les contrôleurs 120 pour réaliser un basculement 135a depuis le mode MD1 vers le mode MD2 et/ou pour réaliser un basculement 135b depuis le mode MD2 vers le mode MD1 (figure 8). A cette fin, on peut par exemple contrôler, au moyen du convertisseur 132, la tension de grille sur la grille G du MOSFET 130 utilisé en tant que contrôleur 120 (par exemple 120a/120b, 122 et/ou 124) de sorte à réaliser le ou les basculement souhaités entre MD1 et MD2.

Il est par exemple possible de configurer le ou les contrôleurs 120 pour alterner entre les modes MD1 et MD2 (figure 8). Les durées pendant lesquelles les modes MD1 et MD2 sont effectifs peuvent être réglées pour être égales ou différentes.

Selon un exemple, la modulation F1 des signaux de sortie SG1 est réalisée en fonction d'un mode d'imagerie implémenté par le pulseur 101 pour piloter le dispositif émetteur 20.

Selon un exemple, la modulation F1 des signaux de sortie SG1 est réalisée en fonction d'un type du dispositif émetteur 20.

Selon un exemple, la modulation F1 des signaux de sortie SG1 est réalisée en fonction de paramètres utilisateurs du dispositif émetteur 20 (par exemple via un contrôle par logiciel pouvant éventuellement mettre en oeuvre une intelligence artificielle).

Selon un exemple, la modulation F1 des signaux de sortie SG1 est réalisée en fonction d'au moins l'un quelconque parmi les critères précités, à savoir un mode d'imagerie implémenté par le pulseur 101, un type du dispositif émetteur 20 et des paramètres utilisateurs du dispositif émetteur 20.

Comme indiqué ci-avant, le concept de la présente divulgation peut s'appliquer de façon analogue à une pluralité de pulseurs 101 du système de pilotage 100. Ainsi, selon un exemple illustré schématiquement en figure 9, le dispositif transducteur 20 comprend une pluralité de transducteurs 22 pilotés chacun par un pulseur 101 respectif. Dans ce cas, la modulation F1 des signaux de sortie SG1 est réalisé indépendamment par canal CN entre chaque transducteur 22 et le pulseur 101 respectif. Pour ce faire, la modulation F1 est réalisée sur les signaux de sortie SG1 de chaque pulseur 101 par un (ou au moins un) contrôleur 120 comme précédemment décrit pour un pulseur 101 donné.

Plus précisément, chaque transducteur 22 peut être piloté par un pulseur respectif 101 au travers d'une chaîne de transmission permettant la transmission de signaux de sortie (ou signaux électriques) SG1 depuis le pulseur 101 jusqu'au transducteur 22. Chacune de ces chaînes de transmission constitue un canal CN (figure 9) au travers duquel un pulseur 101 peut piloter un transducteur 22 respectif du dispositif transducteur 20 et ainsi contrôler les ondes W1 émises par ledit transducteur dans le milieu M. Comme illustré à titre d'exemple en figure 9, n pulseurs notés 101-1 à 101-n peuvent ainsi être configurés pour piloter des transducteurs respectifs 22-1 à 22-n au travers de canaux respectifs CN1 à CNn (n étant un entier supérieur ou égal à 2). Il est ainsi possible de réaliser une modulation F1 par canal et donc d'adapter indépendamment les signaux de sortie SG1 transmis à chaque transducteur 22.

Cette modulation F1 par canal permet avantageusement de piloter le dispositif transducteur 20 de façon encore plus précise et flexible. Cela permet avantageusement en particulier d'adapter les signaux de sortie SG1 délivrés à chaque transducteur 22, indépendamment des autres transducteurs 22. Par exemple, dans le cadre d'une calibration du système de pilotage 100 ou du système SY1, la puissance transmise à chaque transducteur 22 peut être optimisée. Si une même modulation impacte plusieurs canaux/transducteurs à la fois, la calibration doit prendre en compte les variations de caractéristiques de tous les transducteurs du groupe concerné et un compromis doit alors être trouvé pour respecter les limites et spécificités de chaque transducteur. La modulation par canal permet avantageusement d'affiner la calibration ou configuration de chaque transducteur séparément.

A noter qu'il est possible de réaliser une telle modulation F1 dans tous les canaux (ou chaînes de transmission) du système SY1 ou seulement dans une sous-partie d'entre eux, selon l'effet recherché. Il est ainsi possible de configurer les différents transducteurs 22 du système de façon flexible, par exemple en modulant les signaux de sortie SG1 délivrés à au moins un transducteur 22 tandis que les signaux de sortie SG1 délivrés à au moins un autre transducteur 22 ne sont pas modulés.

Selon un exemple, il est par exemple possible de configurer la modulation F1 par canal pour piloter les transducteurs 22 du dispositif transducteur 20 selon une fonction dite d'apodisation.

Conventionnellement, une telle fonction d'apodisation peut être réalisée en faisant varier l'amplitude des signaux de sortie SG1 transmis aux transducteurs 22 en fonction de la position des transducteurs par rapport à un point focal (une zone de focalisation) dans le milieu M, ce qui permet d'adapter l'énergie délivrée dans le milieu E et en particulier de mieux focaliser cette énergie en un point (ou région) d'intérêt dans le milieu M. Conventionnellement, avec des pulseurs toutou-rien / carrés, on réalise cette variation d'amplitude en modifiant le rapport cyclique du signal de sortie du pulseur (ratio entre le temps passé à l'état "haut" (V+ ou V-) et la période du signal). Ainsi, on modifie effectivement la valeur efficace de la puissance transmise au transducteur. Cependant, modifier le rapport cyclique modifie également le contenu spectral du signal transmis, ce qui limite la qualité de l'apodisation, qui n'impacte que l'amplitude du signal et non son spectre.

Selon un exemple, la modulation F1 est configurée pour modifier l'amplitude des signaux SG1 transmis en fonction de la position du transducteur 22, et ce tout en conservant un même rapport cyclique entre tous les canaux, ce qui permet de conserver le même contenu spectral pour chaque transducteur 22. L'effet de l'apodisation est donc meilleur, notamment sur la qualité d'image dans le cas d'une application d'imagerie, puisque le principe théorique de l'apodisation est mieux respecté.

Selon un exemple de la présente divulgation, le système 100 peut être configuré pour réaliser une modulation F1 par canal sur une pluralité de transducteurs 22 du dispositif émetteur 20, de sorte que plus un transducteur 22 du dispositif 20 est éloigné d'un point focal d'intérêt dans le milieu M et plus la tension du signal de sortie SG1 envoyé sur ce transducteur 22 est faible (même forme d'onde mais avec une tension décroissante). En contrôlant la modulation F1 par canal, on peut avantageusement piloter les transducteurs 22 pour émettre des ondes selon une fonction d'apodisation, et ce avec plus de précision et de flexibilité. Par exemple, on peut configurer la modulation F1 pour qu'au moins un transducteur 22 soit bridé en tension (voire désactivé) pour limiter l'énergie délivrer par ce transducteur dans le milieu M. On peut ainsi adapter le pilotage des transducteurs 22 et éviter efficacement des problèmes de mauvaise focalisation des ondes W1 et d'échos W2 indésirables.

Dans le procédé de pilotage décrit ci-avant en référence notamment à la figure 6, la modulation F1 des signaux de sortie SG1 du pulseur 101 peut être réalisée de diverses manières, par exemple de façon déterministe ou quelconque, en prédéfinissant par exemple la manière dont les signaux SG1 doivent être modulés (sans tenir compte des performances réelles du système) ou en adaptant la modulation F1 en fonction du comportement ou des caractéristiques du système de pilotage 100, voire du système SY1 dans son ensemble. Dans les exemples de réalisation qui suivent, la modulation F1 des signaux de sortie SG1 est adaptée en fonction du comportement de la chaîne de transmission s'étendant du pulseur 101 au transducteur 22.

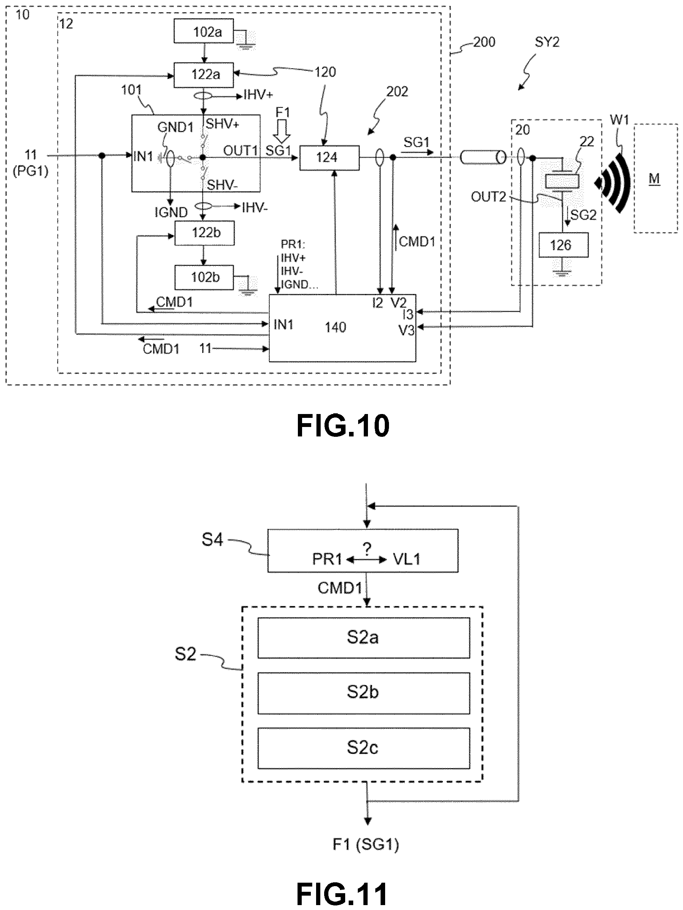

Des exemples de réalisation du système de pilotage 100 (figures 4-9), noté par la suite 200, qui mettent en oeuvre le concept de la présente divulgation, vont à présent être décrits ci-après en référence aux figures 10-15. Sauf indication contraire, les éléments décrits ci-avant en référence au système de pilotage 10 s'appliquent de façon analogue au système de pilotage 100. Comme déjà indiqué, on suppose dans ce qui suit que le système 200 est un système d'imagerie ultrasonore bien que d'autres implémentations soient possibles. Par souci de simplification de l'exposé, certains éléments décrits ci-avant en référence aux figures 1-9 ne sont pas à nouveau décrits en détail ci-après.

Sauf indication contraire, on considère dans ce qui suit par souci de simplification de l'exposé de la divulgation que le système de pilotage 200 utilise un unique pulseur 101 pour piloter un transducteur 22 du dispositif transducteur 20, bien que des variantes soient possibles où une pluralité de pulseurs 101 sont utilisés pour piloter un ou des transducteurs 22 du dispositif 20. Les modes de réalisation de la présente divulgation s'appliquent de façon analogue au pilotage d'un ou de transducteurs 22 par une pluralité de pulseurs 101.

Plus précisément, la figure 10 représente schématiquement selon un exemple le système de pilotage (ou système de commande) 200 comprenant l'unité de commande 12 configurée pour piloter le dispositif transducteur 20, à savoir un dispositif à ultrasons dans cet exemple. Le système de pilotage 200 et le dispositif transducteur 20 forment ensemble un système noté SY2.

Le système de pilotage 200 (figure 10) diffère principalement du système 100 (figure 4) en ce que la modulation F1 des signaux de sortie SG1 émis par le pulseur 101 est adaptée en fonction du comportement d'une chaîne de transmission 202 s'étendant du pulseur 101 au transducteur 22. Pour ce faire, le système de pilotage 200 comprend une unité de commande 140 configurée pour mesurer au moins un paramètre de fonctionnement PR1 de la chaîne de transmission 202 au travers de laquelle le pulseur 101 envoie les signaux de sortie SG1 au transducteur 22. L'unité de commande 140 est en outre configurée pour contrôler le ou les contrôleurs 122 inclus dans le système de pilotage 200, ou plus généralement dans le système SY2, en fonction du ou des paramètres PR1 mesurés. L'unité de commande 140 est par exemple configurée pour envoyer des commandes CMD1 aux contrôleurs 120 pour contrôler la modulation F1 des signaux de sortie SG1 au cours du temps. On peut ainsi améliorer le pilotage du transducteur 22 en ajustant la modulation F1 au comportement du système.

La chaîne de transmission 202 comprend un ensemble de composants matériels, du système SY2, s'étendant depuis le pulseur 101 au transducteur 22 et pouvant comprendre au moins un composant intermédiaire ou autre participant dans la transmission des signaux électriques SG1 depuis le pulseur 101 pour piloter le transducteur 22. Cette chaîne de transmission 202 comprend en particulier le pulseur 101 et le transducteur 22, voire d'autres composants tels qu'une liaison électrique reliant le pulseur 101 au transducteur 22.

Cette chaîne de transmission 202 peut se caractériser par un ou des paramètres de fonctionnement PR1 dont le nombre et la nature peuvent varier selon le cas d'usage.

L'unité de commande 140 peut en particulier comprendre, ou utiliser, de quelconques moyens appropriés pour mesurer le ou les paramètres de fonctionnement PR1 de la chaîne de transmission 202. Des exemples de mise en oeuvre de ces moyens de mesure sont décrits ultérieurement en référence notamment aux figures 11-15.

Selon un exemple, le ou les paramètres de fonctionnement PR1 mesurés par l'unité de commande 140 caractérise au moins l'un parmi un état du pulseur et un état du transducteur 22 (ou plus généralement du dispositif transducteur 20).

Le ou les paramètres de fonctionnement PR1 mesurés peuvent comprendre au moins un paramètre électrique (tension, courant ou puissance par exemple) et/ou au moins un paramètre physique tel qu'une température, une champ électrique, un champ magnétique ou une pression acoustique.

Selon un exemple, le ou les paramètres de fonctionnement PR1 mesurés par l'unité de commande 140 comprennent au moins l'un parmi :
- une tension V2 en sortie du pulseur 101 ;
- une tension d'alimentation (HV- et/ou HV+) du pulseur 101 ;
- une tension de masse du pulseur 101 ;
- une tension V3 aux bornes du dispositif émetteur 20 ;
- une tension en réception du transducteur 22 ;
- un courant I2 en sortie du pulseur 101 ;
- un courant d'alimentation (IHV+, IHV-) du pulseur 101 ;
- un courant de masse IGND du pulseur 101 ;
- un courant I3 circulant dans le dispositif émetteur 20 ;
- une température du pulseur 101 ;
- une température du dispositif émetteur 20 ;
- un champ électrique émis par le pulseur 101 ;
- un champ électrique émis par le dispositif émetteur 20 ;
- un champ magnétique émis par le pulseur 101 ;
- un champ magnétique émis par le dispositif émetteur 20 ;
- une pression acoustique émise par le dispositif émetteur 20 ; et
- une pression acoustique reçue par le dispositif émetteur 20.

Le ou les paramètres de fonctionnement PR1 mesurés peuvent correspond à l'un quelconque des paramètres précités ou à une quelconque combinaison d'au moins deux paramètres parmi ceux-ci.

La tension en réception mentionnée ci-dessus désigne la tension d'un signal de réponse, dit signal « écho », générée par le transducteur 22 en réponse aux ondes W2 (figure 1) reçues du milieu M, par exemple en réponse aux ondes W1 (stimuli) émises par le dispositif transducteur 20.

Un procédé de pilotage mis en oeuvre par le système de pilotage 200 tel que précédemment décrit (figure 10) est à présent décrit conjointement à la figure 11 selon des modes de réalisation particuliers. A cet effet, le système de pilotage 200 (ou plus généralement le système SY2) peut exécuter le programme d'ordinateur PG1. Le système 100 peut exécuter des instructions d'au moins un programme d'ordinateur, dont par exemple le programme PG1 et/ou un programme (non représenté) mis en oeuvre par l'unité de commande 140.

Selon un exemple, l'unité de commande 140 opère en réponse à des instructions de l'unité de traitement 11 (figure 10).

Au cours d'une étape S4 de mesure (figure 11), l'unité de commande 140 mesure un ou des paramètres de fonctionnement PR1 de la chaîne de transmission 202 au travers de laquelle le pulseur 101 envoie les signaux de sortie SG1 au transducteur 22. On suppose par la suite qu'une pluralité de paramètres PR1 sont mesurés bien que des variantes soient possibles où un unique paramètre PR1 est mesuré.

Au cours d'une étape S2 de modulation, les signaux de sortie SG1 du pulseur 101 sont modulés par un (ou au moins un) contrôleur 120 comme déjà décrit en référence à la figure 6. En particulier, comme déjà décrit, cette modulation F1 peut être réalisée par un contrôleur 120 selon au moins l'un parmi :
a) un contrôle (S2a) d'une tension d'alimentation (V+ et/ou V-) du pulseur 101 par un contrôleur d'alimentation 122a et/ou 122b connecté à une borne d'alimentation IHV+ et/ou IHV- du pulseur 101 ;
b) un contrôle (S2b) de signaux de sortie SG1 par un contrôleur de sortie 124 connecté en sortie OUT1 du pulseur 101 ; et
c) un contrôle (S2c) de signaux de retour SG2 du transducteur 22 par un contrôleur de retour 126 connecté sur une borne de retour OUT2 du transducteur 22.

Le procédé de pilotage mis en oeuvre par le système 200 (figure 10) diffère de celui précédemment décrit en référence au système 100 (figure 4) en ce que la modulation F1 des signaux de sortie SG1 est réalisée (S2) en fonction des paramètres de fonctionnement PR1 mesurés en S4. En d'autres termes, la modulation F1 réalisée en S2 est en fonction du résultat des mesures réalisées au préalable en S4. On peut ainsi avantageusement adapter les signaux SG1 émis par le pulseur 101 pour piloter le transducteur 22 en fonction du comportement de la chaîne de transmission 202, rendant ainsi le pilotage plus adaptatif et donc plus performant.

Selon un exemple, l'unité de commande 140 compare (S4) les paramètres de fonctionnement PR1 avec respectivement des valeurs seuils, par exemple pour déterminer si ces paramètres PR1 excèdent lesdits valeurs seuils. La modulation F1 réalisée en S2 peut alors être fonction d'un résultat de ces comparaisons. Cette comparaison peut être réalisée pour un ou une pluralité de paramètres de fonctionnement PR1 selon le cas considéré. Il est ainsi possible de vérifier si les limites de divers composants (ou parties) de la chaîne de transmission 202 sont respectées (limite maximale de tension, limite maximale de courant, limite maximale de température, etc.).

Selon un exemple, l'unité de commande 140 commande au moins un contrôleur d'alimentation 122a/122b de sorte que :
- la tension d'alimentation correspondante est régulée à une première valeur au cours du contrôle S2a (figure 11) si un (au moins un) paramètre de fonctionnement PR1 n'excède pas une valeur seuil VL1 respective, et
- le contrôle S2a comprend une adaptation de la tension d'alimentation depuis la première valeur vers une deuxième valeur, différente de la première valeur, si le (ou ledit au moins un) paramètre de fonctionnement PR1 excède ladite valeur seuil VL1 respective. Selon un exemple, les première et deuxième valeurs sont des valeurs non nulles.

On peut ainsi adapter la tension d'alimentation du pulseur 101, et donc ses signaux de sortie SG1 envoyés au transducteur 22, en fonction du niveau de la tension aux bornes d'alimentation du pulseur 101. Si besoin, on peut par exemple brider le pulseur 101 en puissance en définissant une deuxième valeur (non nulle) inférieure à la première valeur (fonctionnement du pulseur 101 en mode dégradé ou bridé). Selon un exemple, on peut désactiver le pulseur 101 si la valeur seuil VL1 est atteinte.

Selon un exemple, l'unité de commande 140 commande les contrôleurs 120 pour faire converger (ou tendre) au moins un paramètre de fonctionnement PR1 mesuré vers une valeur de référence (ou consigne), par exemple pour faire tendre un rendement de puissance du dispositif émetteur 20 vers une consigne de rendement donné (rendement entre la puissance en entrée délivrée par les régulateurs 102a/102b et la puissance en sortie délivrée par le ou les transducteurs).

Selon un exemple, la modulation F1 des signaux de sortie SG1 est réalisée (S2, figure 11) par asservissement de l'un au moins parmi les contrôles S2a, S2b et S2c en fonction du ou des paramètres de fonctionnement PR1 mesurés en S4. On peut ainsi adapter dynamiquement les signaux de sortie SG1, par exemple en temps réel, de façon à réaliser un pilotage réactif et performant du transducteur 22.

Selon un exemple, la mesure S4 (figure 11) du ou des paramètres de fonctionnement PR1 est réalisée au cours d'une calibration de la chaîne de transmission 202. Cette calibration 202 peut par exemple être réalisée une seule fois (par exemple au stade de la production en usine) ou de façon répétée au cours du temps (par exemple à des démarrages ou mises sous tension du système de pilotage 200, ou encore à chaque fois qu'un nombre prédéfini d'utilisations du système de pilotage 200 est atteint ou à chaque fois qu'une durée prédéfinie d'utilisation du système de pilotage 200 est atteinte).

La modulation F1 est par exemple réalisée après la calibration précitée, par exemple en réponse à au moins une commande CMD1 de l'unité de commande 140. Ce ou ces commandes CMD1 peuvent par exemple être déterminée en fonction du ou des paramètres de fonctionnement. Ces commandes CMD1 peuvent ou non être asservies au cours du temps comme déjà décrit. Il est ainsi possible d'adapter la modulation F1 des signaux SG1, et donc d'ajuster le pilotage du dispositif transducteur 20, en fonction du comportement de l'ensemble. Ceci permet notamment d'adapter la configuration de systèmes de pilotage pour compenser des disparités physiques ou structurelles entre les composants entre les systèmes, ce qui permet d'assurer une meilleure uniformité des performances et de la fiabilités entre les systèmes.

Comme déjà indiqué, le concept de la présente divulgation peut s'appliquer de façon analogue à une pluralité de pulseurs 101 du système de pilotage 200. Ainsi, selon un exemple, le dispositif transducteur 20 comprend une pluralité de transducteurs 22 pilotés chacun par un pulseur 101 respectif. Dans ce cas, la modulation F1 des signaux de sortie SG1 est réalisé indépendamment par canal entre chaque transducteur 22 et le pulseur 101 respectif. Pour ce faire, la modulation F1 est réalisée sur les signaux de sortie SG1 de chaque pulseur 101 par un (ou au moins un) contrôleur 120 comme précédemment décrit pour un pulseur 101 donné. Chaque transducteur 22 peut ainsi être piloté par un pulseur respectif 101 au travers d'une chaîne de transmission 202 permettant la transmission de signaux de sortie (ou signaux électriques) SG1 depuis le pulseur 101 jusqu'au transducteur 22. Chacune de ces chaînes de transmission 202 constitue un canal au travers duquel un pulseur 101 peut piloter un transducteur 22 respectif du dispositif transducteur 20 et ainsi contrôler les ondes W1 émises par ledit transducteur dans le milieu M. Il est ainsi possible de réaliser une modulation F1 par canal qui est fonction d'au moins un paramètre de fonctionnement PR1 mesuré au cours de l'étape S4 (figure 11) de mesure, de façon à adapter indépendamment les signaux de sortie SG1 transmis à chaque transducteur 22.

Selon un exemple, l'unité de commande 140 mesure (S4 ; figure 11) au moins un paramètre de fonctionnement PR1 de chaînes de transmission 202 au travers desquelles chaque traducteur 22 du dispositif transducteur 20 reçoit des signaux de sortie SG1 d'un pulseur respectif 101. Sur détection qu'un (ou au moins un) paramètre de fonctionnement PR1 d'une chaîne de transmission 202 satisfait un critère prédéfini (par exemple dépassement d'une valeur seuil VL1), la modulation F1 des signaux de sortie SG1 émis depuis le pulseur 101 de la chaîne de transmission 202, vers le transducteur 22 de ladite chaîne, est adaptée en conséquence (par exemple modification d'une configuration en puissance et/ou de forme d'onde selon laquelle les signaux de sortie SG1 sont émis). Cette modulation F1 asservie par canal permet de piloter le dispositif transducteur 20 de façon encore plus précise et flexible. La modulation individuelle par canal permet avantageusement de compenser des différences ou variations de caractéristiques entre canaux ou transducteurs, afin de limiter les disparités de comportement entre transducteurs et ainsi uniformiser leurs performances.

Selon un exemple, sur détection d'une modification d'une tension ou d'un courant dans une chaîne de transmission 202 indiquant une chute ou panne du transducteur 22 de ladite chaîne, l'unité de commande 140 contrôle le ou les contrôleurs 120 pour réduire ou brider la puissance des signaux de sortie SG1 émis pour piloter le transducteur 22 de la chaîne de transmission 202. On peut ainsi reconfigurer dynamiquement un transducteur 22 présentant une anomalie, de sorte que ce transducteur continue à émettre des ondes W1, mais limitées ou bridées en puissance, en direction du milieu M.

Des exemples de mises en oeuvre des moyens de mesure des paramètres de fonctionnement PR1 sont à présent décrits à titre purement illustratif de la présente divulgation.

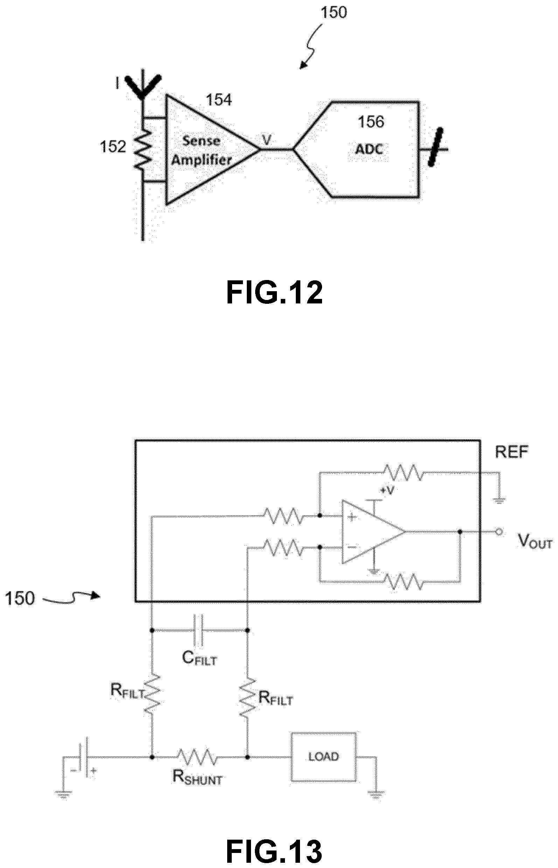

La figure 12 représente schématiquement des moyens de mesure (ou unité de mesure) 150 utilisés par l'unité de commande 140 (figure 10) pour mesurer un courant I dans le système SY2, tel que l'un au moins des courants précités (courant I2 en sortie du pulseur 101, courant d'alimentation IHV+ et/ou IHV- du pulseur 101, courant de masse IGND, etc.). Comme illustré, ces moyens de mesure 150 comprennent par exemple un amplificateur opérationnel 154 dont les deux entrées sont connectées aux bornes d'une résistance 152 au travers de laquelle circule un courant I que l'on souhaite mesurer. Cet amplificateur 154 génère en sortie une tension V représentative du courant I circulant dans la résistance 152. Cette tension V peut ensuite être convertie par exemple par un convertisseur ADC noté 156 (analogique vers numérique) afin que l'unité de commande 140 puisse suivre l'évolution du courant I concerné.

La figure 13 représente schématiquement un exemple de réalisation des moyens de mesure 150 permettant la mesure d'un courant I dans le système SY2.

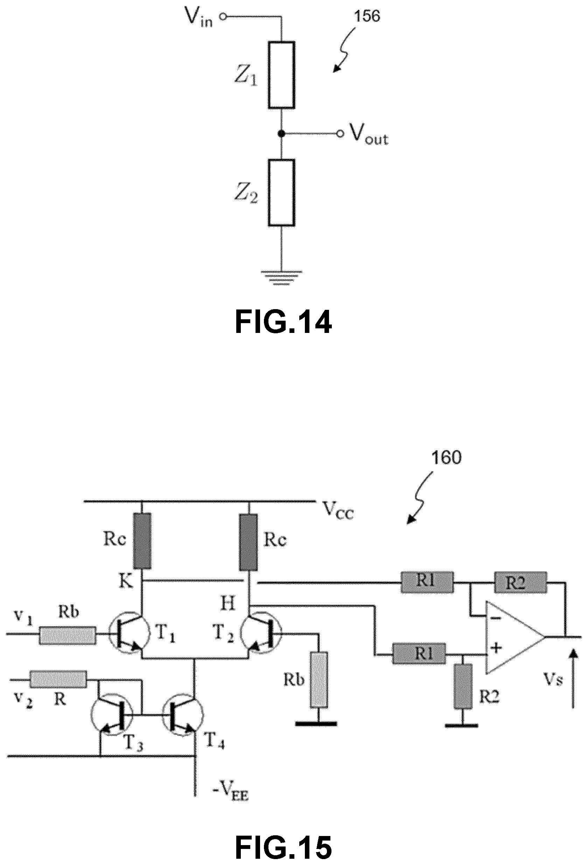

La figure 14 représente schématiquement des moyens de mesure (ou unité de mesure) 156 utilisés par l'unité de commande 140 (figure 10) pour mesurer une tension Vin dans le système SY2, tel que l'une au moins des tensions précitées (tension d'alimentation HV- et/ou HV+, tension V2 en sortie du pulseur 101, tension V3 aux bornes du dispositif émetteur 20, etc.). Ces moyens de mesure 156 forment un pont diviseur à partir de deux impédances Z1 et Z2. A partir d'une mesure de la tension Vout et des impédances Z1 et Z2, on peut en déduire la tension Vin.

La figure 15 représente schématiquement des moyens de mesure (ou unité de mesure) 160 utilisés par l'unité de commande 140 (figure 10) pour mesurer une puissance, telle que la puissance délivrée à au moins l'une des bornes d'alimentation SHV+/SHV- ou la puissance des signaux de sortie SG1. Comme représenté, ces moyens de mesure 160 comprennent un circuit dans lequel des tensions V1 et V2 sont reçues en entrée, dans lequel V1 est une tension acquise dans le système SY2 et V2 étant une tension image d'un courant du système SY2. Ce circuit permet de multiplier les tensions V1 et V2 afin d'obtenir en sortie une tension Vs représentative d'une puissance (où R, Rb, Rc, R1 et R2 sont des résistances et où T1, T2, T3 et T4 sont des transistors).

Comme le comprend l'homme du métier, les moyens de mesure utilisés par l'unité de commande 140 peuvent être adaptés au cas par cas, en fonction notamment du type des paramètres de fonctionnement PR1 à mesurer. A titre d'exemple, un hydrophone peut être utilisé pour mesure une pression acoustique au niveau du dispositif émetteur 20. Un capteur à effet hall peut être utilisé pour mesurer un champ magnétique (ou une variation d'un tel champ) dans le système SY2, par exemple au niveau du pulseur 101 ou du dispositif émetteur 20. Un capteur (ou une sonde) de champ électrique peut également être utilisé pour mesurer un champ électrique dans le système SY2.

Selon un exemple particulier, le procédé de pilotage mis en oeuvre par le système de pilotage 100 ou 200 comprend au moins l'une quelconque parmi :
- une génération d'une alerte si ledit au moins un paramètre de fonctionnement PR1 mesuré (S4, figure 11) satisfait un critère prédéfini ; et
- une estimation, à partir dudit au moins un paramètre de fonctionnement PR1 mesuré, d'une prédiction de vieillissement du dispositif émetteur 20.

Les étapes ci-dessus peuvent être réalisées par exemple par l'unité de commande 140. Outre l'asservissement ou l'adaptation de la modulation F1 des signaux de sortie SG1 (figures 10-11), il est ainsi possible d'utiliser les mesures des paramètres de fonctionnement PR1 pour générer des alertes et/ou faire de la prédiction de vieillissement du dispositif émetteur, ce qui permet d'améliorer notamment l'expérience utilisateur et d'éviter une mauvaise utilisation du système.

Comme le comprend l'homme du métier, tous les modes de réalisation et variantes décrits ci-avant dont certains ont été simplifiés à dessein pour faciliter les explications, ne constituent que des exemples non limitatifs de mise en oeuvre de la présente divulgation. En particulier, l'homme du métier pourra envisager une quelconque adaptation ou combinaison des modes de réalisation et variantes décrits ci-avant, afin de répondre à un besoin particulier.

La présente divulgation ne se limite donc pas aux exemples de réalisation décrits ci-avant mais s'étend notamment à un procédé de pilotage qui inclurait des étapes secondaires sans pour cela sortir de la portée de la présente divulgation. Il en serait de même d'un système de pilotage pour la mise en oeuvre d'un tel procédé.

## Revendications

1. Procédé de pilotage d'un dispositif émetteur (20) comprenant un transducteur (22) pour émettre des ondes, ledit procédé comprenant :
- modulation (S2) de signaux de sortie (SG1) émis depuis un pulseur (101) pour piloter le transducteur, ladite modulation étant réalisé par un contrôleur selon au moins l'un parmi :
a) un contrôle (S2a) d'une tension d'alimentation (V+, V-) du pulseur par un contrôleur d'alimentation (122a, 122b) connecté à une borne d'alimentation (SHV+, SHV-, GND1) du pulseur ; et
c) un contrôle (S2c) de signaux de retour (SG2) du transducteur (22) par un contrôleur de retour (126) connecté sur une borne de retour (OUT2) du transducteur.

2. Procédé selon la revendication 1, dans lequel au cours du contrôle a), le contrôleur d'alimentation (122a, 122b) module une puissance délivrée au pulseur (101) sur la borne d'alimentation à partir d'une tension statique délivrée par un régulateur de tension.

3. Procédé selon l'une quelconque des revendications précédentes, dans lequel la borne d'alimentation (SHV+, SHV-) du pulseur à laquelle est connectée le contrôleur d'alimentation (122a, 122b) est l'une quelconque parmi : une borne d'alimentation positive (SHV+), une borne d'alimentation négative (SHV+-) et une borne de masse (GND1) du pulseur.

4. Procédé selon l'une quelconque des revendications précédentes, dans lequel la modulation est réalisée au moyen d'un dit contrôleur d'alimentation (122a, 122b), la modulation étant configurée pour causer une variation au cours du temps de la tension d'alimentation (V+, V-) du pulseur (101) selon au moins deux valeurs non nulles distinctes.

5. Procédé selon l'une quelconque des revendications précédentes, dans lequel la borne de retour (OUT2) du transducteur (22) est connectée en série avec une masse du dispositif émetteur (20).

6. Procédé selon l'une quelconque des revendications précédentes, dans lequel la modulation (S2) des signaux de sortie est réalisée à une fréquence de modulation Fm supérieure ou égale à une fraction 1/P de la fréquence de tir Ft à laquelle le transducteur (20) émet des ondes (W1) en réponse au signaux de sortie (SG1) du pulseur (101), P étant un entier égal à 10.

7. Procédé selon l'une quelconque des revendications précédentes, dans lequel le dispositif émetteur (20) comprend une pluralité de transducteurs (22) pilotés chacun par un pulseur (101) respectif, la modulation de signaux étant réalisé indépendamment par canal entre chaque transducteur et le pulseur respectif.

8. Procédé selon l'une quelconque des revendications précédentes, dans lequel le procédé comprend :
- mesure (S4) d'au moins un paramètre de fonctionnement (PR1) d'une chaîne de transmission (202) au travers de laquelle le pulseur (101) envoie les signaux de sortie (SG1) au transducteur (22) ;
dans lequel la modulation des signaux de sortie est réalisée en fonction dudit au moins paramètre de fonctionnement (PR1) mesuré.

9. Procédé selon la revendication 8, ledit au moins un paramètre de fonctionnement (PR1) comprenant au moins l'un parmi :
- une tension en sortie du pulseur ;
- la tension d'alimentation du pulseur (V+, V-) ;
- une tension de masse du pulseur ;
- une tension aux bornes du dispositif émetteur (V3) ;
- une tension en réception du transducteur (22) ;
- un courant (I2) en sortie du pulseur ;
- un courant d'alimentation du pulseur (IHV+, IHV-) ;
- un courant de masse (IGND) du pulseur ;
- un courant (I3) circulant dans le dispositif émetteur ;
- une température du pulseur ;
- une température du dispositif émetteur ;
- un champ électrique émis par le pulseur ;
- un champ électrique émis par le dispositif émetteur ;
- un champ magnétique émis par le pulseur ;
- un champ magnétique émis par le dispositif émetteur ;
- une pression acoustique émise par le dispositif émetteur ; et
- une pression acoustique reçue par le dispositif émetteur.

10. Procédé selon la revendication 8 ou 9, dans lequel le procédé comprend :
- comparaison dudit au moins un paramètre de fonctionnement (PR1) avec respectivement une valeur seuil (VL1) ;
la modulation (S2) des signaux de sortie (SG1) étant fonction d'un résultat de ladite comparaison.

11. Procédé selon la revendication 10, dans lequel le contrôleur d'alimentation régule la tension d'alimentation à une première valeur au cours du contrôle a) si ledit au moins un paramètre de fonctionnement n'excède pas une valeur seuil respective, et
dans lequel le contrôle a) comprend une adaptation de la tension d'alimentation depuis la première valeur vers une deuxième valeur, différente de la première valeur, si ledit au moins un paramètre de fonctionnement excède ladite valeur seuil respective.

12. Procédé selon l'une quelconque des revendications 8 à 11, dans lequel la mesure (S4) dudit au moins un paramètre de fonctionnement (PR1) est réalisée au cours d'une calibration de la chaîne de transmission (202),
dans lequel la modulation est réalisée après la calibration en réponse à au moins une commande (CMD1) déterminée en fonction dudit au moins un paramètre de fonctionnement (PR1).

13. Procédé selon une combinaison quelconque des revendications précédentes comprenant la revendication 7, le procédé comprenant :
- mesure d'au moins un paramètre de fonctionnement (PR1) de chaînes de transmission au travers desquelles chaque traducteur (22) reçoit des signaux de sortie (SG1) d'un pulseur (101) respectif ; et
- sur détection qu'un paramètre de fonctionnement d'une chaîne de transmission satisfait un critère prédéfini, adaptation de la modulation des signaux de sortie émis vers le transducteur (22) de ladite chaîne de transmission.

14. Procédé selon l'une quelconque des revendications précédentes, dans lequel ledit procédé est appliqué à l'imagerie médicale par ultrasons.

15. Programme d'ordinateur (PG1) comportant des instructions pour l'exécution des étapes d'un procédé selon l'une quelconque des revendications 1 à 14 lorsque ledit programme est exécuté par un système de pilotage (100 ; 200) pilotant un dispositif transducteur (20).

16. Système de pilotage (100, 200) d'un dispositif (20) émetteur d'ondes comprenant un transducteur (22) pour émettre des ondes, ledit système comprenant :
- un pulseur (101) ; et
- un contrôleur (120) configuré pour moduler des signaux de sortie (SG1) émis depuis le pulseur pour piloter le transducteur, ledit contrôleur comprenant au moins l'un parmi :
a) un contrôleur d'alimentation (122a, 122b), connecté à une borne d'alimentation (SHV+, SHV-, GND1) du pulseur, configuré pour contrôler une tension d'alimentation du pulseur ; et
c) un contrôleur de retour (126), connecté à une borne de retour (OUT2) du transducteur, configuré pour contrôler des signaux de retour (SG2) du transducteur.
